(19) Europäisches Patentamt
European Patent Office
Office européen des brevets

(11) **EP 3 115 430 B1**

(12) **EUROPEAN PATENT SPECIFICATION**

(45) Date of publication and mention
of the grant of the patent:
**05.08.2020 Bulletin 2020/32**

(21) Application number: **15758144.8**

(22) Date of filing: **03.03.2015**

(51) Int Cl.:
*C09J 175/04* (2006.01)      *C08G 18/02* (2006.01)
*C08G 18/76* (2006.01)      *C08G 18/38* (2006.01)
*C08G 18/30* (2006.01)      *C08G 18/18* (2006.01)
*C08G 18/12* (2006.01)      *C08G 18/09* (2006.01)
*C07D 251/32* (2006.01)      *B32B 7/12* (2006.01)
*B32B 27/32* (2006.01)      *B32B 15/20* (2006.01)
*B32B 15/085* (2006.01)      *B32B 37/12* (2006.01)
*C09J 7/29* (2018.01)

(86) International application number:
**PCT/JP2015/056255**

(87) International publication number:
**WO 2015/133496 (11.09.2015 Gazette 2015/36)**

(54) **LAMINATE ADHESIVE, METHOD FOR MANUFACTURING LAMINATE FILM, LAMINATE FILM, AND RETORT POUCH MATERIAL**

LAMINATKLEBSTOFF, VERFAHREN ZUR HERSTELLUNG VON KASCHIERFOLIE, KASCHIERFOLIE UND RETORTENBEUTELMATERIAL

ADHÉSIF DE LAMINATION, FILM DE LAMINATION AINSI QUE PROCÉDÉ DE FABRICATION DE CELUI-CI, ET MATÉRIAU POUR SACHET STÉRILISABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2014 JP 2014041952**
**04.03.2014 JP 2014041957**

(43) Date of publication of application:
**11.01.2017 Bulletin 2017/02**

(73) Proprietor: **Mitsui Chemicals, Inc.**
**Minato-ku**
**Tokyo 105-7122 (JP)**

(72) Inventors:
• **IMAI, Akihiro**
**Sodegaura-shi**
**Chiba**
**2990265 (JP)**
• **MATSUKI, Hirokazu**
**Sodegaura-shi**
**Chiba 299-0265 (JP)**
• **YOSHIDA, Tsutomu**
**Sodegaura-shi**
**Chiba 299-0265 (JP)**
• **NAKASHIMA, Tatsuya**
**Sodegaura-shi**
**Chiba 299-0265 (JP)**
• **MORIYA, Toshiaki**
**Sodegaura-shi**
**Chiba 299-0265 (JP)**

(74) Representative: **Hoffmann Eitle**
**Patent- und Rechtsanwälte PartmbB**
**Arabellastraße 30**
**81925 München (DE)**

(56) References cited:
JP-A- H08 269 428      JP-A- 2000 351 953
JP-A- 2000 351 953      JP-A- 2001 240 839
JP-A- 2002 155 260      JP-A- 2002 155 260
JP-A- 2004 115 681      JP-A- 2005 161 691
JP-A- 2008 239 911      JP-A- 2013 129 105
JP-A- 2014 015 619      JP-A- 2014 088 533

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a laminate adhesive, a method for producing a laminate film, a laminate film, and a retort pouch material. In particular, the present invention relates to a laminate adhesive, a method for producing a laminate film in which the laminate adhesive is used, and a laminate film produced from the method, and a retort pouch material including the laminate film.

BACKGROUND ART

As a wrapping material used in various industrial fields, a laminate film has been known. To be specific, a laminate film produced by laminate processing, for example, a plastic film, metal foil such as aluminum, a metal deposited film, and a silica deposited film with an adhesive has been known.

For the adhesive used for such laminate films, a two-component curable polyurethane adhesive, that is, a two-component curable polyurethane adhesive used by combining a curing agent including a polyisocyanate component and a main component including a polyol component is known.

To be more specific, for example, an adhesive for lamination including a curing agent including a trimethylolpropane adduct of xylylenediisocyanate and a silane coupling agent, and a main component including polyesterpolyol is known, and furthermore, a composite film produced by allowing a plurality of films to adhere using the adhesive for lamination, and maturing it at 50°C for 3 days is known (e.g., ref: Patent Document 1 (Example 1)).

Furthermore, a composite film produced as follows has been known: using an adhesive for dry lamination containing polyester polyurethane polyol, polyester polyol, polyisocyanate including a trimethylol propane adduct of xylylenediisocyanate, and a silane coupling agent; allowing a plurality of films to adhere using the adhesive for dry lamination; and maturing it at 40°C for 2 days or at 40°C for 4 days (e.g., Patent Document 2 (Example 1)).

Citation List

Patent Document

Patent Document 1: Japanese Unexamined Patent Publication No. 2003-113359
Patent Document 2: Japanese Unexamined Patent Publication No. 2000-154365

JP 2002-155260A and JP 2000-351953A relate with adhesives for dry laminations used for wrapping, such as foodstuffs, a beverage, and drugs. The adhesives comprise a polyester polyol and a polyisocyanate hardening agent, which may be a trimer of xylylene diisocyanate.

SUMMARY OF THE INVENTION

PROBLEM TO BE SOLVED BY THE INVENTION

Meanwhile, in the field of composite films, further improvements in interfacial adhesion properties, acid resistance, and content resistance are demanded.

Furthermore, when a laminate adhesive that requires heating and maturing at a temperature of 40°C or more is used in production of composite films, it involves more costs and time for composite film production, and also when the heating and curing are insufficient, it may cause delamination at the time of retorting.

An object of the present invention is to provide a laminate adhesive that has excellent interfacial adhesion properties, acid resistance, and content resistance , can ensure excellent adhesiveness even if matured without heating, and can suppress delamination at the time of heat processing; a method for producing a laminate film in which the laminate adhesive is used; a laminate film produced by the method; and a retort pouch material including the laminate film.

MEANS FOR SOLVING THE PROBLEM

A laminate adhesive of the present invention contains a curing agent including a polyisocyanate component and a main component including a polyol component, wherein the polyisocyanate component contains an isocyanurate derivative of xylylenediisocyanate, and in the chromatogram when the isocyanurate derivative of xylylenediisocyanate is subjected to gel permeation chromatograph measurement, the area percentage of a peak area having peak top between the polystyrene-based molecular weight of 400 to 1000 relative to the total peak area is 30% or more and 85% or less.

In the laminate adhesive of the present invention, it is preferable that in the chromatogram when the isocyanurate derivative of xylylenediisocyanate is subjected to gel permeation chromatograph measurement, the area percentage of a peak area having peak top between the polystyrene-based molecular weight of 400 to 1000 relative to the total peak area is 35% or more and 80% or less.

In the laminate adhesive of the present invention, the polyisocyanate component has an isocyanurate derivative of xylylenediisocyanate content of 20 mass% or more.

In a method for producing a laminate film of the present invention, a plurality of films are allowed to adhere using the above-described laminate adhesive.

In the method for producing a laminate film of the present invention, it is preferable that the adhered film is matured without heating.

A laminate film of the present invention includes a plurality of films, wherein the plurality of films are allowed to adhere using the above-described laminate adhesive.

A retort pouch material of the present invention includes the above-described laminate film.

Effects of the Invention

The laminate adhesive, the method for producing a laminate film in which the laminate adhesive is used, the laminate film produced by the method, and the retort pouch material including the laminate film of the present invention have excellent interfacial adhesion properties, acid resistance, and content resistance , and furthermore, can ensure excellent adhesiveness even if matured without heating, and can suppress delamination at the time of heat processing.

BRIEF DESCRIPTION OF THE DRAWINGS

FIG. 1 shows a gel permeation chromatogram of the isocyanurate derivative of xylylenediisocyanate of Production Example 3.
FIG. 2 shows a gel permeation chromatogram of the isocyanurate derivative of xylylenediisocyanate of Production Example 8.

DESCRIPTION OF EMBODIMENTS

The laminate adhesive of the present invention contains a curing agent including a polyisocyanate component and a main component including a polyol component, and preferably, consists of a curing agent including a polyisocyanate component and a main component including a polyol component.

The polyisocyanate component contains, as an essential component, an isocyanurate derivative of xylylenediisocyanate.

The isocyanurate derivative of xylylenediisocyanate is a trimer of xylylenediisocyanate, and is produced by subjecting xylylenediisocyanate to isocyanurate-forming reaction in the presence of an isocyanurate-forming catalyst.

Xylylenediisocyanate includes structural isomers of 1,2-xylylenediisocyanate (o-xylylenediisocyanate (o-XDI)), 1,3-xylylenediisocyanate (m-xylylenediisocyanate (m-XDI)), and 1,4-xylylenediisocyanate (p-xylylenediisocyanate (p-XDI)).

These xylylenediisocyanates may be used singly or in combination of two or more. For the xylylenediisocyanate, preferably 1,3-xylylenediisocyanate and 1,4-xylylenediisocyanate, more preferably 1,3-xylylenediisocyanate is used.

In the present invention, the isocyanurate-forming catalyst is not particularly limited as long as it is a catalyst that activates isocyanurate formation, and examples thereof include tertiary amines such as triethylamine, tributylamine, triethylenediamine, and a secondary amine copolymer (e.g., polycondensate of secondary amines such as dialkylamine, and a monomer that is copolymerizable with secondary amines (e.g., phenol, formaldehyde, etc.)); mannich base such as 2-dimethylaminomethylphenol and 2,4,6-tris (dimethylaminomethyl) phenol; hydroxides of tetraalkylammoniums such as tetramethylammonium, tetraethylammonium, tetrabutylammonium, trimethylbenzylammonium, and tributylbenzylammonium, and organic weak acid salt thereof; hydroxides of trialkylhydroxyalkylammoniums such as trimethylhydroxypropylammonium (also called: N-(2-hydroxypropyl)-N,N,N-trimethylammonium), trimethylhydroxyethylammonium, triethylhydroxypropylammonium, and triethylhydroxyethylammonium, and organic weak acid salt thereof; metal salt (e.g., salt of alkali metal, magnesium salt, tin salt, zinc salt, lead salt, etc.) of alkylcarboxylic acids such as acetic acid, caproic acid, octylic acid, myristic acid, and naphthenic acid; metal chelate compounds of β-diketone such as aluminum acetylacetone and lithium acetylacetonate; Friedel-Crafts catalysts such as aluminum chloride and boron trifluoride; various organometallic compounds such as titaniumtetrabutyrate and tributylantimonyoxide; aminosilyl-group-containing compounds such as hexamethylsilazane; and hetero substituted organic phosphorus compounds such as tetrabutylphosphonium hydrogendifluoride.

These isocyanurate-forming catalysts may be used singly or in combination of two or more.

For the isocyanurate-forming catalyst, preferably, hydroxide of tetraalkylammonium and hydroxide of trialkylhydroxyalkylammonium are used, and more preferably, hydroxide of tetraalkylammonium is used, even more preferably, hydroxide of trimethylbenzylammonium and hydroxide of tetrabutylammonium are used.

When the above-described catalysts are used as the isocyanurate-forming catalyst, xylylenediisocyanate can be subjected to isocyanurate formation with a particularly excellent reaction rate, and therefore production efficiency is excellent.

The mixing ratio of the isocyanurate-forming catalyst (solid content) relative to 100 parts by mass of xylylenediisocyanate is, for example, 0.001 parts by mass (phr) or more, preferably 0.005 parts by mass (phr) or more, more preferably 0.01 parts by mass (phr) or more, further preferably 0.012 parts by mass (phr) or more, particularly preferably 0.015 parts by mass (phr) or more, and for example, 0.1 parts by mass (phr) or less, preferably 0.06 parts by mass (phr) or less, more preferably 0.05 parts by mass (phr) or less, further preferably 0.03 parts by mass (phr) or less, particularly preferably 0.025 parts by mass (phr) or less.

Then, in this method, xylylenediisocyanate is blended with the isocyanurate-forming catalyst at the above-described mixing ratio, and the mixture is heated to cause isocyanurate-forming reaction.

The reaction conditions for the isocyanurate-forming reaction are as follows: under inert gas atmosphere such as nitrogen gas and normal pressure (atmospheric pressure), the reaction temperature (maximum temperature reached) of, for example, 20°C or more, preferably more than 40°C, more preferably 45°C or more, further preferably 60°C or more, particularly preferably 70°C or more, and for example, 90°C or less, preferably 80°C or less, more preferably 77°C or less, further preferably 75°C or less. The reaction time is, for example, 30 minutes or more, preferably 60 minutes or more, more preferably 120 minutes or more, further preferably 300 minutes or more, and for example, 720 minutes or less, preferably 600 minutes or less, more preferably 480 minutes or less.

In the above-described reaction, to adjust the isocyanurate formation, the organic phosphite described in for example, Japanese Unexamined Patent Publication No. S 61-129173 can be blended as a promoter.

For the organic phosphite, aliphatic organic phosphite and aromatic organic phosphite are used.

Examples of the aliphatic organic phosphite include alkyl monophosphites such as triethyl phosphite, tributyl phosphite, tris (2-ethylhexyl) phosphite, tridecyl phosphite, trilauryl phosphite, tris (tridecyl) phosphite, and tristearyl phosphite; di, tri, or tetra phosphites derived from aliphatic polyhydric alcohols such as distearyl-pentaerythrityl-diphosphite, di·dodecyl·pentaerythritol·diphosphite, di·tridecyl·pentaerythritol·diphosphite, and tripentaerythritol·tri phosphite; and furthermore, alicyclic poly phosphites such as a hydrogenated bisphenol A phosphite polymer (molecular weight 2400 to 3000), and tris (2,3-dichloropropyl) phosphite.

Examples of the aromatic organic phosphite include aryl monophosphites such as triphenyl phosphite, tris (nonylphenyl) phosphite, tris (2,4-di-t-butylphenyl) phosphite, diphenyldecyl phosphite, and diphenyl (tridecyl) phosphite; di, tri, or tetra phosphite derived from aromatic polyhydric alcohol such as dinonylphenyl·pentaerythritol·diphosphite, tetraphenyl·tetra·tridecyl·pentaerythrityl·tetra phosphite, and tetraphenyl·dipropylene glycol·diphosphite; and furthermore, diphosphites derived from bisphenol compounds such as di·alkyl having 1 to 20 carbon atoms·bisphenol A·diphosphite, 4,4'-butylidene-bis(3-methyl-6-t-butylphenyl-di·tridecyl) phosphite.

These organic phosphites may be used singly or in combination of two or more.

For the organic phosphite, preferably, aromatic organic phosphite is used, more preferably, di, tri, or tetra phosphite derived from aromatic polyhydric alcohol is used, even more preferably, tetraphenyl·dipropylene glycol·diphosphite is used.

The mixing ratio of the organic phosphite relative to 100 parts by mass of the xylylenediisocyanate is, for example, 0.01 parts by mass (phr) or more, preferably 0.03 parts by mass (phr) or more, and for example, 0.1 parts by mass (phr) or less, preferably 0.07 parts by mass (phr) or less.

By blending the above-described organic phosphite as the promoter, reaction velocity and reaction rate can be improved, and gellation can be suppressed.

In the above-described reaction, stabilizers including a hindered phenol antioxidant such as 2,6-di(tert-butyl)-4-methylphenol (also called: dibutylhydroxytoluene, hereinafter may be referred to as BHT.), IRGANOX 1010, IRGANOX 1076, IRGANOX 1135, and IRGANOX 245 (all manufactured by Ciba Japan K.K., trade name) can be added at a suitable ratio.

Furthermore, in the above-described reaction, as necessary, a known reaction solvent can be blended, and furthermore, a known catalyst deactivator (e.g., phosphoric acid, monochloroacetic acid, dodecylbenzenesulfonic acid, p-toluenesulfonic acid, benzoyl chloride, etc.) can be added at arbitrary timing.

Then, after the completion of reaction, unreacted xylylenediisocyanate can be removed, as necessary, by a known method.

To be specific, after the completion of the above-described isocyanurate-forming reaction, from the produced reaction mixture liquid of the isocyanurate composition including isocyanurate derivative of xylylenediisocyanate and xylylenediisocyanate monomer, the unreacted xylylenediisocyanate monomer can be removed, for example, by a known method such as distillation including thin film distillation (Smith distillation), and extraction.

In the present invention, when thin film distillation is conducted after completion of the isocyanurate-forming reaction of xylylenediisocyanate, the yield (distillation yield) of isocyanurate derivative produced by the thin film distillation is a mass of isocyanurate derivative of xylylenediisocyanate relative to the mass of the reaction mixture liquid, and is, for example, 16 mass% or more, preferably 25 mass% or more, more preferably 40 mass% or more, and for example, 64 mass% or less, preferably 60 mass% or less.

The distillation yield of the isocyanurate derivative of xylylenediisocyanate can be obtained by calculating percentage of the mass of the isocyanurate derivative of xylylenediisocyanate relative to the mass of reaction mixture liquid in conformity with Examples described later.

In the above-described reaction, as necessary, alcohols can be blended. That is, isocyanurate derivative can be modified with alcohols.

In the present invention, examples of alcohols include, aliphatic alcohol and aromatic alcohol, and preferably, aliphatic alcohol is used.

Examples of the aliphatic alcohol include monohydric aliphatic alcohol, dihydric aliphatic alcohol, trihydric aliphatic alcohol, and aliphatic alcohol having four or more hydroxyl groups.

Examples of the monohydric aliphatic alcohol include straight chain monohydric aliphatic alcohol and branched monohydric aliphatic alcohol.

Examples of the straight chain monohydric aliphatic alcohol include methanol, ethanol, n-propanol, n-butanol, n-pentanol, n-hexanol, n-heptanol, n-octanol, n-nonanol, n-decanol, n-undecanol, n-dodecanol (laurylalcohol), n-tridecanol, n-tetradecanol, n-pentadecanol, n-hexadecanol, n-heptadecanol, n-octadecanol (stearyl alcohol), n-nonadecanol, and eicosanol.

Examples of the branched monohydric aliphatic alcohol include isopropanol (also called: isopropyl alcohol, IPA), isobutanol (also called: isobutyl alcohol, IBA), sec-butanol, tert-butanol, isopentanol, isohexanol, isoheptanol, isooctanol, 2-ethylhexanol (also called: 2-ethylhexyl alcohol, 2-EHA), isononanol, isodecanol, 5-ethyl-2-nonanol, trimethylnonyl alcohol, 2-hexyldecanol, 3,9-diethyl-6-tridecanol, 2-isoheptyl isoundecanol, 2-octyldodecanol, and other branched alkanol (C (number of carbon, the same applies to the following) 5 to 20).

Examples of the dihydric aliphatic alcohol include straight chain dihydric aliphatic alcohols such as ethylene glycol, 1,3-propanediol (1,3-PG), 1,4-butyleneglycol, 1,5-pentanediol, 1,6-hexanediol, 1,4-dihydroxy-2-butene, diethylene glycol, triethylene glycol, dipropylene glycol, and other straight chain alkane (C 7 to 20) diol; branched dihydric aliphatic alcohols such as 1,2-propanediol, 1,3-butyleneglycol (also called: 1,3-butanediol), 1,2-butyleneglycol, neopentyl glycol, 3-methyl-1,5-pentanediol (MPD), 2,2,4-trimethyl-1,3-pentanediol (TMPD), 3,3-dimethylolheptane, 2,6-dimethyl-1-octene-3,8-diol, and other branched alkane (C 7 to 20) diol; alicyclic dihydric aliphatic alcohols such as 1,3- or 1,4-cyclohexanedimethanol and their mixture, 1,3- or 1,4-cyclohexanediol and their mixture, and hydrogenated bisphenol A.

Examples of the trihydric aliphatic alcohol include glycerin and trimethylolpropane.

Examples of the aliphatic alcohol with having four or more hydroxyl groups include tetramethylolmethane, D-sorbitol, xylitol, and D-mannitol.

The molecular structure of these alcohols are not particularly limited as long as one or more hydroxy group is present in the molecule, and as long as excellent effects of the present invention are not hindered, and for example, an ester group, an ether group, a cyclohexane ring, and an aromatic ring can also be present in the molecule. Examples of the alcohols include an ether group-containing monohydric alcohol of an addition polymerization product (random and/or block polymer of two or more types of alkylene oxides) of the above-described monohydric alcohol and alkylene oxide (e.g., ethyleneoxide, propyleneoxide, etc.), and an ester group-containing monohydric alcohol of an addition polymerization product of the above-described monohydric alcohol and lactone (e.g., ε-caprolactone, δ-valerolactone, etc.).

These alcohols may be used singly or in combination of two or more.

For the alcohols, preferably, aliphatic alcohol is used, and more preferably mono or di hydric aliphatic alcohol is used, and more preferably, dihydric aliphatic alcohol is used.

For the aliphatic alcohol, preferably, aliphatic alcohol having 1 to 20 carbon atoms, more preferably, aliphatic alcohol having 4 to 20 carbon atoms, even more preferably, aliphatic alcohol having 4 to 15 carbon atoms are used.

For the aliphatic alcohol, preferably, branched mono and di hydric aliphatic alcohols are used, more preferably, branched dihydric aliphatic alcohol is used.

In the present invention, for the aliphatic alcohol, particularly preferably 1,3-butanediol is used.

The alcohols are blended so that the average functional group in the isocyanurate derivative of xylylenediisocyanate is 2 or more, and the mixing ratio of the alcohols relative to 100 parts by mass of the xylylenediisocyanate is, for example, 0.3 parts by mass or more, preferably 1.0 parts by mass or more, more preferably 1.5 parts by mass or more, and for example, 11 parts by mass or less, preferably 8.0 parts by mass or less, more preferably 7.0 parts by mass or less, further preferably 5.0 parts by mass or less, particularly preferably 3.0 parts by mass or less.

When the mixing ratio of the alcohols is within the above-described range, laminate adhesive with excellent adhesiveness can be produced.

In this reaction, xylylenediisocyanate and alcohols are blended at a mixing ratio such that the equivalent ratio (NCO/OH) of isocyanate group of xylylenediisocyanate relative to the hydroxy group of the alcohols is, for example, 5 or more, preferably 10 or more, more preferably 20 or more, further preferably 25 or more, and usually 1000 or less.

In the reaction, within the range that does not hinder excellent effects of the present invention, as necessary, the above-described alcohols can be used in combination with an active hydrogen group-containing compound such as, for example, thiols, oximes, lactams, phenols, and β diketones.

The isocyanurate derivative of xylylenediisocyanate is modified with alcohols by, for example, the following methods: first, xylylenediisocyanate is allowed to react with alcohols, and then after causing isocyanurate-forming reaction in the presence of an isocyanurate-forming catalyst, unreacted xylylenediisocyanate is removed; and for example, first, after only xylylenediisocyanate is subjected to isocyanurate formation with the above-described method, unreacted xylylenediisocyanate is removed and thereafter, the produced polyisocyanurate is allowed to react with alcohols.

Preferably, first, xylylenediisocyanate is allowed to react with alcohols, and then after causing isocyanurate-forming reaction in the presence of an isocyanurate-forming catalyst, unreacted xylylenediisocyanate is removed.

To be specific, in this method, first, xylylenediisocyanate is mixed with alcohols and allowed to react.

The reaction between xylylenediisocyanate and alcohols is urethane-forming reaction (including allophanate formation reaction), and the reaction conditions are as follows: under inert gas atmosphere such as nitrogen gas and normal pressure (atmospheric pressure), the reaction temperature is, for example, room temperature (e.g., 25°C) or more, preferably 40°C or more, and for example, 100°C or less, preferably 90°C or less. The reaction time is, for example, 0.05 hours or more, preferably 0.2 hours or more, and for example, 10 hours or less, preferably 6 hours or less, more preferably 2.5 hours or less.

In the above-described urethane-forming reaction, as necessary, for example, a known urethane-forming catalyst such as amines and an organometallic compound can be added.

Examples of the amines include tertiary amines such as triethylamine, triethylenediamine, bis-(2-dimethylaminoethyl) ether, and N-methylmorphiline; quaternary ammonium salts such as tetraethyl hydroxyl ammonium; imidazoles such as imidazole and 2-ethyl-4-methyl imidazole.

Examples of the organometallic compound include organic tin compounds such as tin acetate, tin octylate, tin oleate, tin laurate, dibutyltindiacetate, dimethyltindilaurate, dibutyltindilaurate, dibutyltin dimercaptide, dibutyltinmaleate, dibutyltindilaurate, dibutyltin dineodecanoate, dioctyltin dimercaptide, dioctyltin dilaurate, and dibutyltin dichloride; organic lead compounds such as lead octanoate and lead naphthenate; organic nickel compounds such as nickel naphthenate; organic cobalt compounds such as cobalt naphthenate; organic copper compounds such as copper octanoate; and organic bismuth compounds such as bismuth octylate and bismuth neodecanoate.

For the urethane-forming catalyst, for example, potassium salts such as potassium carbonate, potassium acetate, and potassium octylate are used.

These urethane-forming catalysts may be used singly or in combination of two or more.

In this method, the produced reaction solution is blended with the isocyanurate-forming catalyst at the above-described mixing ratio, and the reaction product of the xylylenediisocyanate and alcohols is subjected to isocyanurate-forming reaction. The reaction conditions in isocyanurate formation are the same as the above-described. After the completion of reaction, the unreacted xylylenediisocyanate is removed, as necessary, by a known removal method such as distillation.

The isocyanurate derivative of xylylenediisocyanate modified with alcohols can be produced in this manner.

Furthermore, when unreacted xylylenediisocyanate is removed after subjecting only xylylenediisocyanate to isocyanurate formation, and the produced polyisocyanate is allowed to react with alcohols (latter in the above-described methods), isocyanurate derivative of xylylenediisocyanate is allowed to react with alcohols. This reaction as well is urethane-forming reaction, and the reaction conditions are the same as those in the above-described urethane-forming reaction.

The isocyanurate derivative of xylylenediisocyanate modified with alcohols can be produced in this manner as well.

When the isocyanurate derivative of xylylenediisocyanate is modified with alcohols, an allophanate derivative of xylylenediisocyanate may be produced as a by-product. In such a case, the isocyanurate derivative of xylylenediisocyanate contains allophanate derivative of xylylenediisocyanate as a secondary ingredient contained inevitably. In other words, when the isocyanurate derivative of xylylenediisocyanate is modified with alcohols, an isocyanurate composition containing isocyanurate derivative of xylylenediisocyanate and allophanate derivative of xylylenediisocyanate is produced.

In the isocyanurate derivative of xylylenediisocyanate modified with alcohols, the modification amount of alcohols (alcohol modification percentage of isocyanurate derivative) relative to the isocyanurate derivative is, for example, 0.5 mass% or more, preferably 1.0 mass% or more, more preferably 3.0 mass% or more, and for example, 15 mass% or less, preferably 10 mass% or less, more preferably 7.0 mass% or less, further preferably 6.0 mass% or less, particularly preferably 5.0 mass% or less. The modification amount of alcohols (alcohol modification percentage) relative to the isocyanurate derivative can be calculated based on the following formula.

$$\text{Alcohol modification percentage of isocyanurate derivative (mass\%)} =$$

$$(\text{alcohol modification percentage in reaction mixture liquid (mass\%)/distillation}$$

$$\text{yield (mass\%)}) \times 100$$

The alcohol modification percentage in reaction mixture liquid is a modification amount of alcohols relative to the xylylenediisocyanate and isocyanurate derivative in the reaction mixture liquid, and can be calculated as the mixing ratio of the charged mass of the alcohols relative to the charged mass of xylylenediisocyanate.

Generally, the alcohol modification percentage can also be calculated by $^1$H-NMR measurement.

For example, when aliphatic alcohol is used, in $^1$H-NMR measurement (400MHz, solvent $CDCL_3$ (3%), scanning 128 times) of isocyanurate derivative, the peak of 6.5 to 8.0 ppm of benzeneproton is assigned as a peak of xylylenediisocyanate, and the peak of 0.9 to 1.4 ppm of methylproton is assigned as a peak of aliphatic alcohol. Then, their peak area ratio can be calculated as the molar ratio of xylylenediisocyanate to aliphatic alcohol. Then, from the calculated molar ratio, the mass ratio of xylylenediisocyanate and alcohol can be calculated, and alcohol modification percentage can be calculated.

In the present invention, isocyanurate derivative of xylylenediisocyanate not modified with alcohols is defined as a derivative that does not positively contain the active hydrogen group-containing compound such as the above-described alcohols as a modifier, and for example, the modification group (e.g., urethane group, urea group, etc.) based on the active hydrogen group-containing compound (e.g., solvent (e.g., methanol, etc.) of isocyanurate-forming catalyst, moisture, etc.) inevitably mixed in at the time of production of isocyanurate derivative can be contained in the isocyanurate derivative of xylylenediisocyanate.

The isocyanurate derivative of xylylenediisocyanate has an isocyanate group concentration (solid content 100 mass%) of, for example, 10.0 mass% or more, preferably 15.0 mass% or more, more preferably 18.0 mass% or more, and for example, 25.0 mass% or less, preferably 24.0 mass% or less, and preferably 23.0 mass% or less, further preferably 20.0 mass% or less.

The isocyanate group concentration (solid content 100 mass%) of the isocyanurate derivative of xylylenediisocyanate can be obtained in conformity with Examples described later.

In the isocyanurate derivative of xylylenediisocyanate, the isocyanate monomer concentration (unreacted xylylenediisocyanate concentration) is, for example, 2 mass% or less, preferably 1 mass% or less, more preferably 0.5 mass% or less.

The isocyanurate group conversion rate (reaction rate) of xylylenediisocyanate is, for example, 1 mass% or more, preferably 5 mass% or more, more preferably 10 mass% or more, and for example, 50 mass% or less, preferably 45 mass% or less, more preferably 40 mass% or less.

When the isocyanurate derivative of xylylenediisocyanate is not modified with alcohols, the isocyanate group conversion rate (reaction rate) of xylylenediisocyanate is substantially the same as the isocyanurate conversion rate (trimer conversion rate).

Meanwhile, when the isocyanurate derivative of xylylenediisocyanate is modified with alcohols, the isocyanate group conversion rate (reaction rate) of xylylenediisocyanate is a total value of the urethane conversion rate by the alcohols and the isocyanurate conversion rate (trimer conversion rate).

In such a case, urethane conversion rate is, for example, 0.5 mass% or more, preferably 1.5 mass% or more, more preferably 3.0 mass% or more, and for example, 30 mass% or less, preferably 20 mass% or less, more preferably 10 mass% or less, further preferably 8.0 mass% or less.

The isocyanurate conversion rate (trimer conversion rate) is, for example, 15 mass% or more, preferably 17 mass% or more, and for example, 40 mass% or less, preferably 35 mass% or less.

The isocyanurate group conversion rate, urethane conversion rate, and trimer conversion rate of xylylenediisocyanate can be determined by calculating the decrease rate of the isocyanate group concentration in the reaction solution relative to the isocyanate group concentration of xylylenediisocyanate at the time of charging in conformity with Examples described later.

Then, in the present invention, in the chromatogram when the isocyanurate derivative of xylylenediisocyanate

7

is subjected to gel permeation chromatograph measurement, the area percentage of a peak area having a peak top between the polystyrene-based molecular weight of 400 to 1000, preferably 600 to 900 relative to the total peak area of the peak area (hereinafter referred to as trimolecular product area percentage) is 30% or more, preferably 35% or more, more preferably 40% or more, and 85% or less, preferably 80% or less, further preferably 75% or less.

The trimolecular product area percentage can be calculated by measuring the molecular weight distribution of the isocyanurate derivative of xylylenediisocyanate based on the calibration curve of standard polystyrene with a gel permeation chromatograph (GPC) equipped with a refractive index detector (RID), and as a peak area ratio in the obtained chromatogram (chart) in conformity with Examples described later.

When the isocyanurate derivative of xylylenediisocyanate is not modified with alcohols, the trimolecular product area percentage corresponds to mononuclear isocyanurate (to be specific, a compound in which 3 molecules of xylylenediisocyanate form one isocyanurate ring, and the isocyanurate ring is not bonded with other isocyanurate ring, that is, 3 molecules of xylylenediisocyanate via one isocyanurate ring) content.

When the isocyanurate derivative of xylylenediisocyanate is modified with alcohols, the trimolecular product area percentage corresponds to a total amount of the mononuclear isocyanurate content and the allophanate derivative of 3 molecules of xylylenediisocyanate (to be specific, 2 molecules of xylylenediisocyanate are bonded via alcohols (dihydric alcohol), and 1 molecule of xylylenediisocyanate forms allophanate bond relative to the bonded portion (urethane bond), that is, 3 molecules of xylylenediisocyanate having allophanate bond) content.

When the trimolecular product area percentage is within the above-described range, excellent adhesiveness can be ensured.

The polyisocyanate component can contain, as an optional component, other polyisocyanate (polyisocyanate excluding isocyanurate derivative of xylylenediisocyanate).

Examples of the other polyisocyanate include aliphatic polyisocyanate, araliphatic polyisocyanate, aromatic polyisocyanate, and derivatives of their polyisocyanate (excluding isocyanurate derivative of xylylenediisocyanate).

Examples of the aliphatic polyisocyanate include aliphatic diisocyanates such as trimethylenediisocyanate, tetramethylenediisocyanate, hexamethylene diisocyanate (HDI), 1,2-propylenediisocyanate, 1,2-butylenediisocyanate, 2,3-butylenediisocyanate, 1,3-butylenediisocyanate, 2,4,4- or 2,2,4-trimethylhexamethylene diisocyanate, and 2,6-diisocyanatemethylcaproate.

The aliphatic polyisocyanate include alicyclic polyisocyanate.

Examples of the alicyclic polyisocyanate include alicyclic diisocyanates such as 1,3-cyclopentanediisocyanate, 1,4-cyclohexanediisocyanate, 1,3-cyclohexanediisocyanate, 3-isocyanatomethyl-3,5,5-trimethylcyclohexylisocyanate (also called: isophoronediisocyanate (IPDI)), 4,4'-methylenebis(cyclohexylisocyanate)($H_{12}$MDI), methyl-2,4-cyclohexanediisocyanate, methyl-2,6-cyclohexanediisocyanate, bis(isocyanatomethyl) cyclohexane (1,3-or 1,4-bis(isocyanatomethyl) cyclohexane)($H_6$XDI), bis(isocyanatoethyl) cyclohexane (1,3-or 1,4-bis(isocyanatoethyl) cyclohexane), 2,5-or 2,6-bis(isocyanatomethyl) norbornane (NBDI) and a mixture thereof.

Examples of the araliphatic polyisocyanate include araliphatic diisocyanate such as 1,3-or 1,4-xylylenediisocyanate or a mixture thereof (XDI), 1,3-or 1,4-tetramethylxylylenediisocyanate or a mixture thereof, and $\omega,\omega'$-diisocyanato-1,4-diethylbenzene.

Examples of the aromatic polyisocyanate include aromatic diisocyanate such as 2,4-tolylene diisocyanate and 2,6-tolylene diisocyanate, and isomer mixture of these tolylene diisocyanates (TDI); 4,4'-diphenylmethane diisocyanate, 2,4'-diphenylmethane diisocyanate and 2,2'-diphenylmethane diisocyanate, any isomer mixture of these diphenylmethane diisocyanate (MDI); and toluylenediisocyanate, p-phenylene diisocyanate, and naphthalenediisocyanate.

Examples of the polyisocyanate derivative (however, excluding isocyanurate derivative of xylylenediisocyanate) include multimers (e.g., dimers, trimers (e.g., isocyanurate derivative, iminooxadiazinedione derivative), pentamers, heptamers, etc.), allophanate derivatives (e.g., allophanate derivative produced by reaction of polyisocyanate with a low molecular-weight polyol described later, etc.), polyol derivatives (e.g., polyol derivative produced by reaction of polyisocyanate and a low molecular-weight polyol described later (alcohol adduct), etc.), biuret derivatives (e.g., biuret derivative produced by reaction of polyisocyanate with water or amines, etc.), urea derivatives (e.g., urea derivative produced by reaction of polyisocyanate with diamine, etc.), oxadiazinetrione derivatives (e.g., oxadiazinetrione produced by reaction of polyisocyanate with carbon dioxide, etc.), carbodiimide derivatives (carbodiimide derivative produced by decarboxylation condensation reaction of polyisocyanate, etc.), urethodione derivatives, and uretonimine derivatives.

The other polyisocyanate may be used singly or in combination of two or more.

For the other polyisocyanate, preferably, an aliphatic polyisocyanate derivative, an alicyclic polyisocyanate derivative, and an araliphatic polyisocyanate derivative are used, and more preferably, multimers, polyol derivatives and biuret derivatives of hexamethylene diisocyanate, multimers and polyol derivatives of isophorone diisocyanate, polyol derivatives of xylylenediisocyanate, and allophanate derivatives of xylylenediisocyanate are used.

The polyisocyanate component preferably consists of an isocyanurate derivative of xylylenediisocyanate; consists of an isocyanurate derivative of xylylenediisocyanate and a multimer of hexamethylene diisocyanate; consists of an isocyanurate derivative of xylylenediisocyanate and a polyol derivative of hexamethylene diisocyanate; consists of an isocyanurate derivative of xylylenediisocyanate and a biuret derivative of hexamethylene diisocyanate; consists of an isocyanurate derivative of xylylenediisocyanate and a multimer of isophoronediisocyanate; consists of an isocyanurate derivative of xylylenediisocyanate and a polyol derivative of isophoronediisocyanate; consists of an isocyanurate derivative of xylylenediisocyanate and a polyol derivative of xylylenediisocyanate; consists of an isocyanurate derivative of xylylenediisocyanate and an allophanate derivative of xylylenediisocyanate; consists of an isocyanurate derivative of xylylenediisocyanate, a multimers of hexamethylene diisocyanate, and a polyol derivative of isophoronediisocyanate.

In the polyisocyanate component, the isocyanurate derivative of xylylenediisocyanate content relative to the total amount of the polyisocyanate component is 20 mass% or more, preferably 30 mass% or more, and for example, 100 mass% or less.

When the isocyanurate derivative of xylylenediisocyanate content is in the above-described range, even if matured without heating, excellent adhesiveness, heat resistance, hot water resistance, acid resistance, and content resistance can be achieved.

The polyisocyanate component has an isocyanate group concentration of, for example, 10 mass% or more, preferably 13 mass% or more, and for example, 25 mass% or less, preferably 23 mass% or less.

In the curing agent, the polyisocyanate component can be diluted, as necessary, with an organic solvent. That is, the curing agent can contain an organic solvent.

Examples of the organic solvent include ketones such as acetone, methyl ethyl ketone, methylisobutylketone, and cyclohexanone; nitriles such as acetinitrile; alkylesters such as methyl acetate, ethyl acetate, butyl acetate, and isobutyl acetate; aliphatic hydrocarbons such as n-hexane, n-heptane, and octane; alicyclic hydrocarbons such as cyclohexane and methylcyclohexane; aromatic hydrocarbons such as toluene, xylene, and ethylbenzene; glycol etheresters such as methylcellosolveacetate, ethylcellosolveacetate, methylcarbitolacetate, ethylcarbitolacetate, ethylene glycolethyletheracetate, propylene glycolmethyletheracetate, 3-methyl-3-methoxybutylacetate, and ethyl-3-ethoxypropionate; ether such as diethylether, tetrahydrofuran, and dioxane; halogenated aliphatic hydrocarbons such as methyl chloride, methylene chloride, chloroform, carbon tetrachloride, methyl bromide, methylene iodide, and dichloroethane; polar aprotics such as N-methylpyrrolidone, dimethylformamide, N,N'-dimethylacetamide, dimethyl sulfoxide, and hexamethyl phosphor amide.

Examples of the organic solvent further include nonpolar solvents (nonpolar organic solvent), and examples of these nonpolar solvents include low toxicity and solvency nonpolar organic solvent having an aniline point of, for example, 10 to 70°C, preferably 12 to 65°C including aliphatic, naphthene hydrocarbon organic solvent, and vegetable oil represented by turpentine oil.

The nonpolar organic solvent is available as a commercially available product, and examples of the commercially available products include petroleum hydrocarbon organic solvents such as HAWS (manufactured by Shell Chemicals Japan Ltd., aniline point 15°C), Swazol 310 (manufactured by MARUZEN PETROCHEMICAL CO.,LTD., aniline point 16°C), Esso Naphtha No. 6 (manufactured by Exxon Mobil Corporation, aniline point 43°C), Laws (manufactured by Shell Chemicals Japan Ltd., aniline point 43°C), Esso Naphtha No. 5 (manufactured by Exxon Mobil Corporation, aniline point 55°C), and PEGASOL 3040 (manufactured by Exxon Mobil Corporation, aniline point 55°C); and also turpentine oils including methylcyclohexane (aniline point 40°C), ethylcyclohexane (aniline point 44°C), and Gum Turpentine N (manufactured by YASUHARA CHEMICAL CO., LTD., aniline point 27°C).

The organic solvent may be used singly or in combination of two or more.

The polyisocyanate component is mixed with an organic solvent at any proportion.

When the polyisocyanate component is diluted with an organic solvent, the polyisocyanate component concentration is, for example, 20 mass% or more, preferably 30 mass% or more, and for example, 95 mass% or less, preferably 90 mass% or less.

The curing agent has a viscosity at 25°C of, for example, 100 mPa·s or more, for example, 10000 mPa·s or less, preferably 5000 mPa·s or less.

Examples of the polyol component include high molecular weight polyol.

The high molecular weight polyol is a compound having two or more hydroxyl groups and a number average molecular weight of 400 or more, and examples thereof include polyetherpolyol, polyesterpolyol, polyesteramidepolyol, polycarbonatepolyol, polyurethane polyol, epoxy polyol, vegetable oil polyol, polyolefinpolyol, acrylic polyol, and vinyl monomer-modified polyol.

Examples of the polyetherpolyol include polyalkylenepolyol, polytetramethylene ether glycol, and polytrimethyleneetherglycol.

Examples of the polyalkylenepolyol include addition polymerization product (including random and/or block copolymer of two or more alkyleneoxides) of alkyleneoxides such as ethyleneoxide and propyleneoxide using a low molecular-weight polyol or aromatic/aliphatic polyamine to be described later as an initiator.

Examples of the polytetramethylene ether glycol include ring-opening polymerization product produced by cationic polymerization of tetrahydrofuran and noncrystalline polytetramethylene ether glycol produced by copolymerizing the above-described dihydric alcohol to the polymerization unit of tetrahydrofuran.

Furthermore, plant derived polytetramethylene ether glycol in which tetrahydrofuran produced based on a plant derived material such as furfural as a starting material can also be used.

Examples of the polytrimethyleneetherglycol include polyol produced by polycondensation of plant derived 1,3-propanediol.

Examples of the polyesterpolyol include a polycondensate produced by allowing a low molecular-weight polyol (preferably dihydric alcohol) to be described later to react with polybasic acid under known conditions.

Examples of the polybasic acid include saturated oxalic acid, malonic acid, succinic acid, methylsuccinic acid, glutaric acid, adipic acid, 1,1-dimethyl-1,3-dicarboxypropane, 3-methyl-3-ethylglutaric acid, azelaic acid, sebacic acid, and the other aliphatic dicarboxylic acids (having 11 to 13 carbon atoms); unsaturated aliphatic dicarboxylic acids such as maleic acid, fumaric acid, itaconic acid, etc.; aromatic dicarboxylic acids such as orthophthalic acid, isophthalic acid, terephthalic acid, toluenedicarboxylic acid, naphthalenedicarboxylic acid, etc.; alicyclic dicarboxylic acid such as hexahydrophthalic acid, etc.; the other carboxylic acids such as dimer acid, hydrogenated dimer acid, HET acid, etc., and acid anhydrides derived from these carboxylic acids such as oxalic anhydride, succinic anhydride, maleic anhydride, phthalic anhydride, succinic 2-alkyl (C 12 to C 18) anhydride, tetrahydrophtalic anhydride, trimellitic anhydride, and furthermore, halides derived from these carboxylic acids such as oxalic acid dichloride, adipic acid dichloride, and sebacic acid dichloride.

Examples of the polyesterpolyol include vegetable oil-based polyesterpolyol produced by subjecting a low molecular-weight polyol to be described later and hydroxycarboxylic acid such as a hydroxyl group-containing vegetable oil fatty acid (e.g., castor oil fatty acid containing ricinoleic acid, hydrogenated castor oil fatty acid containing 12-hydroxystearic acid, etc.) to condensation reaction under known conditions.

Examples of the polyesterpolyol include polycaprolactonepolyol and polyvalerolactonepolyol produced by subjecting lactones such as ε-caprolactone and γ-valerolactone to ring-opening polymerization using a low molecular-weight polyol (preferably dihydric alcohol) to be described later as an initiator, and also the lactone polyesterpolyol produced by copolymerizing the above-described dihydric alcohol with these.

Examples of the polyesteramidepolyol include polyesteramidepolyol produced by using a low molecular-weight polyamine (e.g., ethylene diamine, propylene diamine, hexamethylenediamine, etc.) as an ingredient in combination in the above-described esterification reaction of polyesterpolyol.

Examples of the polycarbonatepolyol include ring-opening polymerization product of ethylenecarbonate using a low molecular-weight polyol (preferably dihydric alcohol) to be described later as an initiator, and noncrystalline polycarbonatepolyol produced by copolymerizing dihydric alcohol such as 1,4-butanediol, 1,5-pentanediol, 3-methyl-1,5-pentanediol, and 1,6-hexanediol and a ring-opening polymerization product.

The polyurethane polyol can be produced as polyesterpolyurethane polyol, polyetherpolyurethane polyol, polycarbonatepolyurethane polyol, or polyesterpolyetherpolyurethane polyol by allowing the polyesterpolyol, polyetherpolyol and/or polycarbonatepolyol produced as described above to react with the above-described polyisocyanate (including isocyanurate derivative of xylylenediisocyanate. The same applies to the following) so that the equivalent ratio (OH/NCO) of the hydroxyl group relative to the isocyanate group is more than 1.

Examples of the epoxy polyol include epoxy polyol produced by reaction of a low molecular-weight polyol to be described later with polyfunctional halohydrin such as epichlorohydrin and β-methylepichlorohydrin.

Examples of the vegetable oil polyol include hydroxyl group-containing vegetable oil such as castor oil and coconut oil. For example, castor oil polyol, or an ester-modified castor oil polyol produced by reaction of castor oil polyol with polypropylenepolyol is included therein.

Examples of the polyolefinpolyol (polyhydroxyalkane) include polybutadiene polyol and a partially saponified ethylene-vinyl acetate copolymer.

Examples of the acrylic polyol include a copolymer produced by copolymerizing hydroxyl group-containing acrylate and a copolymerizable vinyl monomer that is copolymerizable with hydroxyl group-containing acrylate.

Examples of the hydroxyl group-containing acrylate include 2-hydroxyethyl (meth)acrylate, hydroxypropyl (meth)acrylate, hydroxybutyl (meth)acrylate, 2,2-dihydroxymethylbutyl (meth)acrylate, polyhydroxyalkylmaleate, and polyhydroxyalkylfumarate. Preferably, 2-hydroxyethyl (meth)acrylate is used.

Examples of the copolymerizable vinyl monomer include alkyl (meth)acrylate (having 1 to 12 carbon atoms) such as methyl (meth)acrylate, ethyl (meth)acrylate, propyl (meth)acrylate, isopropyl (meth)acrylate, butyl (meth)acrylate, isobutyl (meth)acrylate, s-butyl (meth)acrylate, t-butyl (meth)acrylate, pentyl (meth)acrylate, isopentyl (meth)acrylate, hexyl (meth)acrylate, isononyl (meth)acrylate, 2-ethylhexyl (meth)acrylate, cyclohexylacrylate, and isobornyl (meth)acrylate; aromatic vinyl monomers such as styrene, vinyltoluene, and α-methylstyrene; vinyl cyanides such as (meth) acrylonitrile; vinyl monomers containing a carboxyl group such as (meth) acrylic acid, fumaric acid, maleic acid, and itaconic acid or its alkylester; alkanepolyol poly (meth)acrylate such as ethylene glycoldi (meth)acrylate, butyleneglycoldi (meth)acrylate, hexanedioldi (meth)acrylate, oligoethylene glycoldi (meth)acrylate, trimethylolpropanedi (meth)acrylate, and trimethylolpropanetri (meth)acrylate; and isocyanate group-containing vinyl monomers such as 3-(2-isocyanate -2-propyl)-α-methylstyrene.

The acrylic polyol can be produced by copolymerizing these hydroxyl group-containing acrylate, and a copolymerizable vinyl monomer in the presence of a suitable solvent and a polymerization initiator.

The acrylic polyol includes, for example, silicone polyol and fluorine polyol.

Examples of the silicone polyol include acrylic polyol in which in the above-described copolymerization of acrylic polyol, a silicone compound containing a vinyl group such as y-methacryloxypropyltrimetoxysilane as a copolymerizable vinyl monomer is blended.

Examples of the fluorine polyol include acrylic polyol in which in the above-described acrylic polyol copolymerization, a fluorine compound including a vinyl group such as tetrafluoroethylene and chlorotrifluoroethylene as a copolymerizable vinyl monomer is blended.

The vinyl monomer-modified polyol can be produced by reaction of the above-described high molecular weight polyol with a vinyl monomer.

For the high molecular weight polyol, preferably, a high molecular weight polyol selected from polyether polyol, polyester polyol, and polycarbonatepolyol is used.

Examples of the vinyl monomer include the above-described alkyl (meth)acrylate, vinyl cyanide, or vinylidene cyanide. These vinyl monomers may be used singly or in combination of two or more. Of these, preferably, alkyl (meth)acrylate is used.

The vinyl monomer-modified polyol can be produced by allowing these high molecular weight polyols and a vinyl monomer in the presence of, for example, a radical polymerization initiator (e.g., persulfate, organic peroxide, azo compound, etc.).

These high molecular weight polyols may be used singly or in combination of two or more.

For the high molecular weight polyol, preferably, polyesterpolyol, polyetherpolyol, and polyurethane polyol are used.

The polyol component can include a low molecular-weight polyol.

The low molecular-weight polyol is a compound having two or more hydroxyl groups and a number average molecular weight of 60 or more and less than 400, and examples thereof include dihydric alcohols such as ethylene glycol, propylene glycol, 1,3-propanediol, 1,4-butyleneglycol, 1,3-butyleneglycol, 1,2-butyleneglycol, 1,5-pentanediol, 1,6-hexanediol, neopentyl glycol, 3-methyl-1,5-pentanediol, 2,2,2-trimethylpentanediol, 3,3-dimethylolheptane, alkane (C 7 to 20) diol, 1,3-or 1,4-cyclohexanedimethanol and a mixture thereof, 1,3-or 1,4-cyclohexanediol and a mixture thereof, hydrogenated bisphenol A, 1,4-dihydroxy-2-butene, 2,6-dimethyl-1-octene-3,8-diol, bisphenol A, diethylene glycol, triethylene glycol, and dipropylene glycol; trihydric alcohols such as glycerin, trimethylolpropane, and triisopropanolamine; tetrahydric alcohols such as tetramethylolmethane (pentaerythritol) and diglycerol; pentahydric alcohols such as xylitol; hexahydric alcohols such as sorbitol, mannitol, allitol, iditol, dulcitol, altritol, inositol, and dipentaerythritol; heptahydric alcohols such as perseitol; and octahydric alcohols such as sucrose.

These low molecular-weight polyols may be used singly or in combination of two or more.

In the polyol component, the high molecular weight polyol content and the low molecular-weight polyol content relative to 100 parts by mass of their total is as follows: the high molecular weight polyol content is, for example, 60 parts by mass or more, preferably 70 parts by mass or more, and for example, 100 parts by mass or less. The low molecular-weight polyol content is, for example, 0 part by mass or more, and for example, 40 parts by mass or less, preferably 30 parts by mass or less.

The polyol component has a number of an average functional group of, for example, 2 or more, and for example, 6 or less, preferably 4 or less.

The polyol component has a number average molecular weight (polystyrene standard calibration) of, for example, 200 or more, preferably 300 or more, and for example, 200000 or less, preferably 100000 or less, more preferably 50000 or less.

The above-described polyol component can be, as necessary, modified with acid.

The polyol component can be modified with acid without particular limitation, and a known method is used. To be specific, for example, acid anhydride such as trimellitic anhydride, phthalic anhydride, maleic anhydride, and pyromellitic anhydride is allowed to react with a terminal hydroxyl group of a polyol component (that is, high molecular weight polyol and/or low molecular-weight polyol). The reaction conditions are suitably set in accordance with the type of the high molecular weight polyol and the type of the acid anhydride.

The polyol component has an acid value of usually 0 mgKOH/g or more, and for example, 280 mgKOH/g or less, preferably 100mg KOH/g or less, and more preferably 50 mgKOH/g.

In the main component, the polyol component may be diluted, as necessary, with an organic solvent. That is, the main component can contain an organic solvent.

Examples of the organic solvent include those organic solvents contained in the above-described curing agent. The organic solvent may be used singly or in combination of two or more.

The polyol component is mixed with an organic solvent at any proportion.

When the polyol component is diluted with an organic solvent, the polyisocyanate component has a concentration of, for example, 20 mass% or more, preferably 30 mass% or more, and for example, 95 mass% or less, preferably 90 mass% or less.

The main component has a viscosity at 25°C of, for example, 100mPa·s or more, for example, 10000mPa·s or less, preferably 5000mPa·s or less.

The laminate adhesive can contain, known additives such as, for example, a silane coupling agent, a compound containing a sulfonamide group, oxygen acid of phosphorus or its derivative, and furthermore, epoxy resin, catalyst, carboxylic acid or its anhydride, an antioxidant, ultraviolet absorber, hydrolysis prevention agent, antifungal agent, thickening agent, plasticizer, pigment, filler, and an antifoaming agent at a suitable proportion.

Examples of the silane coupling agent include any silane coupling agent represented by structural formula R-Si=(X)$_3$ or R-Si=(R')(X)$_2$ (where R represents an organic group having a vinyl group, epoxy group, amino group, imino group, isocyanate group or mercapto group, and R' represents a lower alkyl group, and X represents a methoxy group, ethoxy group or chlorine atom), and chlorosilane such as vinyltrichlorosilane; aminosilanes such as N-β-(aminoethyl)-γ-aminopropyltrimetoxysilane, γ-aminopropyltriethoxysilane, N-β-(aminoethyl)-γ-propylmethyldimethoxysilane, n-(dimethoxymethylsilylpropyl) ethylene diamine, n-(triethoxysilylpropyl) ethylene diamine, and N-phenyl-γ-aminopropyltrimetoxysilane; epoxy silanes such as γ-glycidoxypropyltrimetoxysilane, γ-glycidoxypropyltriethoxysilane, β-(3,4-epoxycyclohexyl) ethyltrimetoxysilane, and di (γ-glycidoxypropyl) dimethoxysilane; vinyl silanes such as vinyltriethoxysilane; and isocyanate silanes such as 3-isocyanate propyltrimetoxysilane and 3-isocyanate propyltriethoxysilane.

These silane coupling agents may be used singly or in combination of two or more.

Examples of the compound containing a sulfonamide group include aromatic sulfonamides and aliphatic sulfonamides.

Examples of the aromatic sulfonamides include benzene sulfonamide, dimethylbenzene sulfonamide, sulfanilamide, o- and p-toluene sulfonamide, hydroxynaphthalene sulfonamide, naphthalene-1-sulfonamide, naphthalene-2-sulfonamide, m-nitrobenzene sulfonamide, and p-chlorobenzene sulfonamide.

Examples of the aliphatic sulfonamides include, for example, methane sulfonamide, N,N-dimethylmethane sulfonamide, N,N-dimethylethane sulfonamide, N,N-diethylmethane sulfonamide, N-methoxymethane sulfonamide, N-dodecylmethane sulfonamide, N-cyclohexyl-1-butanesulfonamide, and 2-aminoethane sulfonamide.

These compounds containing a sulfonamide group may be used singly or in combination of two or more.

Examples of the oxygen acid of phosphorus include phosphoric acids such as hypophosphorous acid, phosphorous acid, orthophosphoric acid, and hypophosphoric acid; and condensed phosphates such as metaphosphoric acid, pyrophosphoric acid, tripolyphosphoric acid, polyphosphoric acid, and ultraphosphoric acid.

Examples of the derivative of oxygen acid of phosphorus include phosphates or condensed phosphates of sodium and potassium, etc.; monoesters such as monomethyl orthophosphate, monoethyl orthophosphate, monopropyl orthophosphate, monobutyl orthophosphate, mono-2-ethylhexyl orthophosphate, monophenyl orthophosphate, monomethyl phosphite, monoethyl phosphite, monopropyl phosphite, monobutyl phosphite, mono-2-ethylhexyl phosphite, and monophenyl phosphite; di and tri esters such as di-2-ethylhexyl orthophosphate, diphenyl orthophosphate, trimethyl orthophosphate, triethyl orthophosphate, tripropyl orthophosphate, tributyl orthophosphate, tri-2-ethylhexyl orthophosphate, triphenyl orthophosphate, dimethyl phosphite, diethyl phosphite, dipropyl phosphite, dibutyl phosphite, di-2-ethylhexyl phosphite, diphenyl phosphite, trimethyl phosphite, triethyl phosphite, tripropyl phosphite, tributyl phosphite, tri-2-ethylhexyl phosphite, and triphenyl phosphite; or mono, di, and tri esters produced from condensed phosphate and alcohols.

These oxygen acids of phosphorus or their derivatives may be used singly or in combination of two or more.

When the laminate adhesive contains the above-described additive, the timing for blending is not particularly limited, and for example, the additive can be blended with one or both of the curing agent and the main component; or can be blended simultaneously at the time of blending the curing agent with the main component; or can be blended separately after blending the curing agent with the main component. The addition proportion is suitably set in accordance with the type of the additive.

The laminate adhesive of the present invention is prepared, for example, as a two-component curable polyurethane resin.

That is, the laminate adhesive of the present invention is used as follows: the curing agent and the main component are prepared separately in advance, and at the time of use, the curing agent and the main component are blended and applied to an adherend.

The mixing ratio of the curing agent to the main component is as follows: the equivalent ratio (NCO/OH) of the isocyanate group in the curing agent relative to the hydroxyl group in the main component is, for example, 0.4 or more, preferably 0.5 or more, and for example, 10 or less, preferably 6 or less.

When the laminate adhesive is used as a two-component curable polyurethane resin (that is, two-component type urethane adhesive), the curing agent and the main component are separately prepared, and therefore a longer pot life is achieved, and by blending the necessary minimum amount of the curing agent and the main component when in use, the adhesive can be used effectively as an adhesive that achieves quick curing and excellent adhesion performance.

The above-described laminate adhesive has excellent interfacial adhesion properties, resistance to heat and moist (hot water resistance), high temperature sterilization suitability (heat resistance), acid resistance, solvent resistance, and content resistance . Therefore, the above-described laminate adhesive is suitably used as an adhesive for producing a laminate film by allowing a plurality of films to adhere (bond), to be specific, as a laminate adhesive for producing a wrapping material in various industrial fields such as food, beverage, medical and pharmaceutical products, and quasi drug.

The present invention includes a laminate film produced by using the above-described laminate adhesive and a production method thereof.

In the method for producing a laminate film of the present invention, the above-described laminate adhesive is used to allow a plurality of films to adhere.

For example, when a curing agent and a main component that are diluted with an organic solvent are used, after mixing the curing agent and the main component, a solvent-type laminator is used to apply the mixture to the surface of the films, and after volatilizing the solvent, the surface for adhesion are bonded together, and thereafter, allowed to mature and cure under normal temperature or while heated. The amount to be applied is, preferably, after solvent volatilization, about 2.0 to 5.0 g/m$^2$.

When a curing agent and a main component that are not diluted with an organic solvent are used, the curing agent and the main component are mixed, and thereafter a no-solvent-type laminator is used to apply the mixture to the surface of the films, the surface for adhesion are bonded together, and thereafter, allowed to mature and cure under normal temperature or while heated. The amount to be applied is, preferably, about 1.0 to 4.0 g/m$^2$.

Examples of the film to be laminated include plastic films such as polyethylene terephthalate, nylon, polyethylene, polypropylene, and polyvinyl chloride: foil of metal such as aluminum; metal deposited film; silica deposited film; alumina deposited film; silica-alumina composite deposited film; and metal films such as stainless steel, iron, copper, and lead.

These films may be used singly or in combination of two or more. The film can be subjected to a known processing as necessary, such as drawing, corona treatment, and coating.

The thickness of the film is suitably set in accordance with type and use, and for example, in the case of plastic film, preferably 5 to 200 $\mu$m.

Then, in this method, the adhered film can be matured (heat-mature) under heat, but can also be matured (non-heat-mature) without heating the adhered film.

The temperature conditions in heat-maturing are, for example, 30°C or more, preferably 40°C or more, and for example, 80°C or less, preferably 60°C or less.

The maturing time is, for example, 12 hours or more, preferably 24 hours or more, and for example, 10 days or less, preferably for 7 days or less.

The temperature conditions in non-heat-maturing are as follows: room temperature (that is, without heating, ambient temperature of the place where maturing takes place), to be specific, for example, -10°C, preferably 0°C or more, more preferably 10°C or more, further preferably 20°C or more, for example, less than 40°C, preferably 35°C or less, more preferably less than 30°C.

The maturing time is, for example, 12 hours or more, preferably 24 hours or more, and for example, 10 days or less, preferably for 7 days or less.

With such a method for producing a laminate film without heating, film shrinkage can be suppressed, and productivity of the laminate film can be improved.

That is, when a laminate adhesive that requires heat-mature in production of a laminate film is used, it takes time to conduct heat to the core of the roll of the composite film, and therefore the portion near the core of the roll is cured insufficiently, and may cause delamination at the time of retorting.

Furthermore, when the laminate film has to be heat-matured, the heating causes the film to shrink, and due to the shrinking, disadvantages may be caused such as, for example, displaced pitch or wrinkles caused at the core of the roll.

Meanwhile, by using the laminate adhesive of the present invention, even if maturing is performed without heating, excellent adhesive strength can be exhibited, and therefore heat-mature is unnecessary, film shrinkage can be suppressed, and thermosetting cannot be insufficient, and therefore delamination can be suppressed. Therefore, laminate film productivity can be improved.

The above-described laminate adhesive, the method for producing a laminate film in which the laminate adhesive is used, and the laminate film produced by the method allows for excellent interfacial adhesion properties, acid resistance, and content resistance , and furthermore, even if maturing is performed without heating, excellent adhesiveness can be ensured, and delamination at the time of heating can be suppressed.

To be specific, a laminate film produced by using such a laminate adhesive of the present invention allows for suppression of delamination at the time of hot water treatment even if high temperature sterilization processing such as, for example, hot water spraying, hot water rotation, or steaming is performed, in which hot water treatment with 100°C or more is performed, and for suppression of interlayer delamination.

The above-described laminate film has excellent content resistance, and is suitably used even for use where charged with for example, acidic food such as vinegar and ketchup, and for example, hygiene products such as detergent, shampoo, and conditioner, and requires adhesive strength after storage.

Therefore, the laminate film obtained by using the above-described laminate adhesive can be suitably used for use where interfacial adhesion properties, heat resistance, hot water resistance, acid resistance, solvent resistance, and content resistance are required, and in particular, used more suitably for a retort pouch material to which heat sterilization at 100°C or more is performed.

The retort pouch material is composed, for example, of a composite film, and to be specific, composed of a composite film produced by laminating a plurality of films with the above-described laminate adhesive.

Examples of the film to be laminated include plastic films such as polyethylene terephthalate, nylon, polyethylene, polypropylene, and polyvinyl chloride; foil of metal such as aluminum; metal deposited film; silica deposited film; alumina deposited film; silica-alumina composite deposited film; and metal films such as stainless steel, iron, copper, and lead. The film has a thickness of, for example, in the case of a plastic film, 5 to 200 $\mu$m.

For the lamination method, a known method is used. To be more specific, for example, the above-described laminate adhesive is applied on a film with a known laminator such as solvent-type laminator and no-solvent-type laminator, and as necessary after volatilizing the solvent, the applied surfaces are bonded, and thereafter, matured and cured. The amount applied is, for example, in the case of solvent-type, after solvent volatilization, about 2.0 to 5.0 g/m$^2$, in the case of no-solvent-type, about 1.0 to 4.0 g/m$^2$.

A retort pouch material composed of a composite film is produced in this manner.

Such a retort pouch material contains an isocyanurate derivative of xylylenediisocyanate as a polyisocyanate component of laminate adhesive, and therefore even if maturing is performed without heating, adhesiveness is excellent, and delamination at the time of heating can be suppressed.

Examples

The specific numeral values such as mixing ratio (content), physical property values, and parameters used in the following can be replaced with the upper limit value (numeral values defined as "or less", and "less than") or lower limit value (numeral values defines as "or more", and "more than") of corresponding mixing ratio (content), physical property values, and parameters in the above-described "DESCRIPTION OF EMBODIMENTS".

The measurement methods used in Examples and Comparative Examples are described below.

<Distillation yield of isocyanurate derivative>

The distillation yield of isocyanurate derivative is determined by measuring the mass of the reaction mixture liquid (liquid before distillation) and the mass of the isocyanurate derivative (liquid after distillation), and calculating the mass ratio of the mass of the isocyanurate derivative relative to the mass of the reaction mixture liquid based on the following formula.

$$\text{Distillation yield of isocyanurate derivative (mass\%)} = (\text{mass of the isocyanurate derivative (g)/mass of the reaction mixture liquid (g)}) \times 100$$

<Modification amount with alcohols relative to isocyanurate derivative (alcohol modification percentage of isocyanurate derivative)>

The modification amount of alcohols (alcohol modification percentage in reaction mixture liquid) in the reaction mixture liquid relative to the xylylenediisocyanate and isocyanurate derivative was calculated as a charged mass of alcohols relative to a charged mass of xylylenediisocyanate.

Furthermore, the modification amount of alcohols (alcohol modification percentage of isocyanurate derivative) relative to the isocyanurate derivative was calculated based on the following formula.

$$\text{Alcohol modification percentage of isocyanurate derivative (mass\%)} = (\text{alcohol modification percentage in reaction mixture liquid (mass\%)/distillation yield (mass\%)}) \times 100$$

<Conversion rate (reaction rate)(%)>

The isocyanate group concentration in the reaction solution (reaction mixture liquid or isocyanurate derivative) was measured in conformity with JIS K-1603-1(2010), and the reduction rate was determined. The isocyanate group conversion rate (reaction rate) was obtained in this manner.

When the isocyanurate derivative of xylylenediisocyanate is not modified with alcohols, the isocyanate group conversion rate is the trimer conversion rate.

When the isocyanurate derivative of xylylenediisocyanate is modified with alcohols, the isocyanate group conversion rate after addition of alcohols and before addition of isocyanurate-forming catalyst is the urethane conversion rate. The isocyanate group conversion rate after addition of the isocyanurate-forming catalyst is the trimer conversion rate.

<Trimolecular product area percentage>

The isocyanurate derivative sample was subjected to gel permeation chromatography (GPC) measurement, and in the obtained chromatogram (chart), the trimolecular product area percentage was obtained from the area percentage of the peak area having peak top between polystyrene-based molecular weight of 400 to 1000 relative to the total peak area.

The trimolecular product area percentage is also the area percentage of the peak area having peak top between retention time of 26.8 minutes to 27.1 minutes relative to the total peak area in the chromatogram (chart) obtained in the device below.

In the GPC measurement, about 0.04 g of a sample was taken and subjected to methylurethane formation with methanol, and then excessive methanol was removed, and 10mL of tetrahydrofuran was added and the sample was dissolved. Then, the prepared solution was subjected to GPC measurement with the following conditions.

(1) Analysis device: Alliance (Waters)
(2) Pump: Alliance 2695 (Waters)
(3) Detector: type 2414 Refractive Index Detector (Waters)
(4) Eluent: Tetrahydrofuran
(5) Separation column: Plgel GUARD + Plgel 5 $\mu$m Mixed-C $\times$ 3 (50 $\times$ 7.5 mm, 300 $\times$ 7.5 mm)

Manufacturer; Polymer Laboratories
Product number; PL 1110-6500

(6) Measurement temperature: 40°C
(7) Flow rate: 1mL/min
(8) Sample injection amount: 100$\mu$L
(9) Analysis device: EMPOWER data processing device (Waters)

·System correction

(1) Standard material name: Polystyrene
(2) Calibration curve making method: using TSK standard Polystyrene having a different molecular weight manufactured by TOSOH, a graph for retention time to molecular weight was made.
(3) Injection amount, injection concentration: 100$\mu$L, 1mg/mL

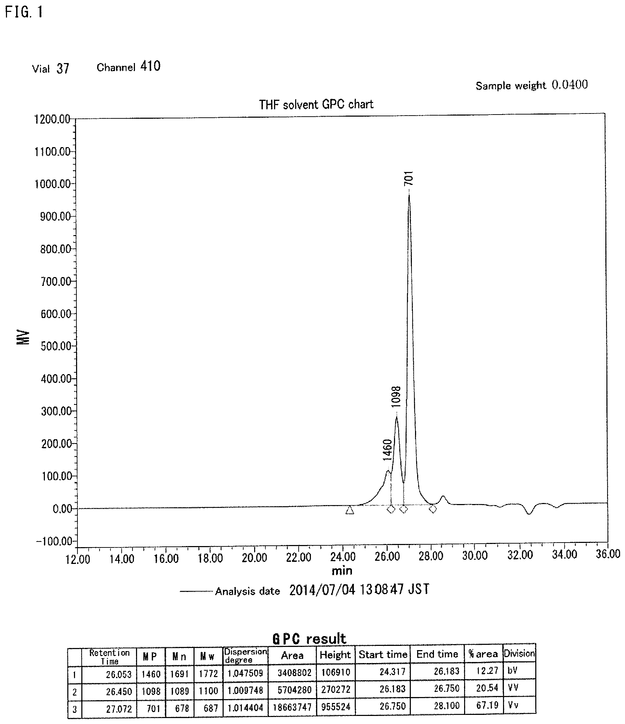

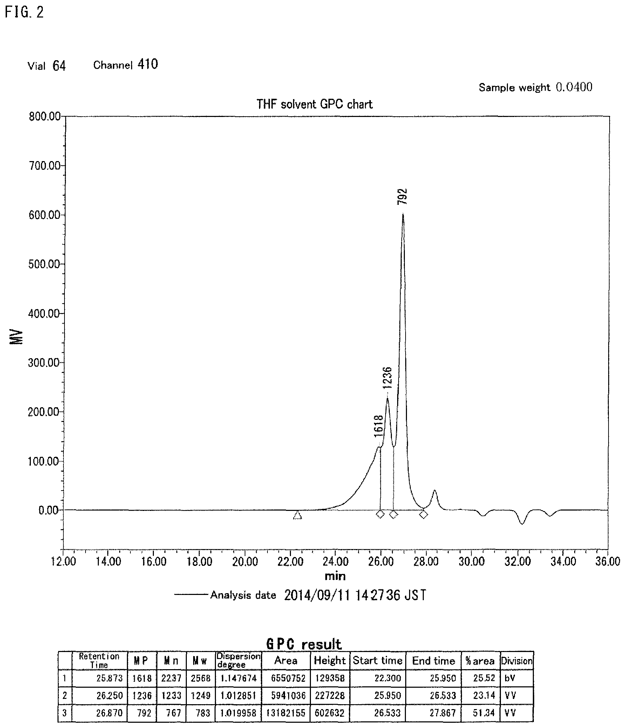

FIG. 1 shows a gel permeation chromatogram of the isocyanurate derivative of xylylenediisocyanate of Production Example 3, and FIG. 2 shows a gel permeation chromatogram of the isocyanurate derivative of xylylenediisocyanate of Production Example 8.

<Isocyanurate derivative of xylylenediisocyanate>

Production Example 1

A reactor equipped with a thermometer, a mixer, a nitrogen inlet tube, and a condenser tube was charged with 100 parts by mass of 1,3-xylylenediisocyanate (manufactured by Mitsui Chemicals, Inc., m-XDI), 0.025 phr of 2,6-di(tert-butyl)-4-methylphenol (also called: dibutylhydroxytoluene, BHT, hindered phenol antioxidant (stabilizer)), and 0.05 phr of tetraphenyl-dipropylene glycol·diphosphite (JPP-100 (trade name, manufactured by Johoku Chemical Co. Ltd.) organic phosphite, promoter) in a nitrogen atmosphere, and to the charged liquid, as an isocyanurate-forming catalyst, 0.032 phr (solid content-based 0.012phr) of solution (37% methanol solution) of tetrabutylammonium hydroxide (TBAOH) was blended, and allowed to react with a reaction start temperature of 60°C for 100 minutes. The maximum temperature reached during reaction was 65°C.

Then, the produced reaction mixture liquid was introduced to a thin film distillation device (temperature 150°C, degree of vacuum 50Pa), and unreacted xylylenediisocyanate was removed, thereby producing an isocyanurate derivative of xylylenediisocyanate. The distillation yield was 9.3 mass%.

The alcohol modification percentage in this reaction was 0 mass%, the isocyanate group conversion rate (that is, trimer conversion rate) was 5.0 mass%, and the trimolecular product area percentage was 88%.

Production Examples 2 to 6

An isocyanurate derivative of xylylenediisocyanate was produced in the same manner as in Production Example 1, except that the formulation and production conditions are changed as shown in Table 1.

Production Example 7

A reactor equipped with a thermometer, a mixer, a nitrogen inlet tube, and a condenser tube was charged with 100 parts by mass of 1,3-xylylenediisocyanate (manufactured by Mitsui Chemicals, Inc., m-XDI), 0.025 phr of 2,6-di (tert-butyl)-4-methylphenol (also called: dibutylhydroxytoluene, BHT, hindered phenol antioxidant), and 0.05 phr of tetraphenyl-dipropylene glycol·diphosphite (JPP-100(trade name, manufactured by Johoku Chemical Co. Ltd.) organic phosphite, promoter) in a nitrogen atmosphere, and to the charged liquid, 1.96 parts by mass of 1,3-butanediol was added, and the temperature of the charged liquid was increased to 75°C to cause urethane-forming reaction. The equivalent ratio (NCO/OH) of isocyanate group of 1,3-xylylenediisocyanate relative to the hydroxy group of 1,3-butanediol was 24.

Then, after allowing the mixture to react at the same temperature for 120 minutes, the temperature was reduced to 60°C. Then, as an isocyanurate-forming catalyst, 0.04 phr (solid content-based 0.015phr) of solution (37% methanol solution) of tetrabutylammonium hydroxide (TBAOH) was blended to cause isocyanurate-forming reaction. The isocyanurate-forming reaction was terminated at 390 minutes from the start of reaction. The maximum temperature reached during reaction was 71°C.

Then, the produced reaction mixture liquid was introduced to a thin film distillation device (temperature 150°C, degree of vacuum 50Pa), and unreacted xylylenediisocyanate was removed, thereby producing a polyisocyanurate composition. The distillation yield was 60.0 mass%.

The alcohol modification percentage in this reaction was, in the reaction mixture liquid (before distillation), 1.96 mass%, in the isocyanurate derivative (after distillation), 3.27 mass%, the isocyanate group conversion rate was 34.2 mass%, the urethane conversion rate was 5.3 mass%, the trimer conversion rate was 28.9 mass%, and the trimolecular product area percentage was 39%.

Production Examples 8 to 9

An isocyanurate derivative of xylylenediisocyanate was produced in the same manner as in Production Example 7, except that the formulation and the production conditions were changed as shown in Table 2.

[Table 1]

| No. | Production Example 1* | Production Example 2 | Production Example 3 | Production Example 4 | Production Example 5 | Production Example 6 |
|---|---|---|---|---|---|---|
| Alcohol modification agent | Not modified | Not modified | Not modified | Not modified | Not modified | Not modified |
| Alcohol modification percentage (wt%) | - | - | - | - | - | - |
| Additive Amount (phr) BHT | 0.025 | 0.025 | 0.024 | 0.025 | 0.025 | 0.024 |

(continued)

| Additive Amount (phr) | | | | | | |
|---|---|---|---|---|---|---|
| JPP-100 | 0.05 | 0.05 | 0.05 | 0.05 | 0.05 | 0.05 |
| Catalyst | | | | | | |
| Type | TBAOH | TBAOH | TBAOH | TBAOH | TBAOH | TBAOH |
| Added Amount (phr) | 0.032 | 0.040 | 0.048 | 0.056 | 0.048 | 0.040 |
| Added Amount solid content base(phr) | 0.012 | 0.015 | 0.018 | 0.021 | 0.018 | 0.015 |
| Reaction Conditions | | | | | | |
| Reaction Start Temperature(°C) | 60 | 60 | 70 | 60 | 60 | 60 |
| Maximum Temperature Reached(°C) | 65 | 77 | 77 | 72 | 65 | 79 |
| Reaction Time (min) | 100 | 140 | 300 | 480 | 510 | 510 |
| Amine equivalent at the time of reaction start | 94.1 | 94.1 | 94.1 | 94.1 | 94.1 | 94.1 |
| NCO% at the time of reaction start | 44.7 | 44.7 | 44.7 | 44.7 | 44.7 | 44.7 |
| Amine equivalent after completion of reaction | 99.1 | 106.8 | 117.1 | 138.1 | 138.8 | 160.5 |
| NCO% after completion of reaction | 42.4 | 39.3 | 35.9 | 30.4 | 30.3 | 26.2 |
| Isocyanate group conversion rate (%) | 5.0 | 11.9 | 19.6 | 31.9 | 32.2 | 41.4 |
| Urethane conversion rate (%) | 0 | 0 | 0 | 0 | 0 | 0 |
| Trimer conversion rate (%) | 5.0 | 11.9 | 19.6 | 31.9 | 32.2 | 41.4 |
| Distillation yield (%) | 9.3 | 22.2 | 31.5 | 57.4 | 54.3 | 63.8 |
| Alcohol modification percentage (wt%) | - | - | - | - | - | - |
| Urethane-formation percentage (mol%) | - | - | - | - | - | - |
| Properties | | | | | | |
| Solid content (%) | 75.1 | 76.1 | 77.2 | 73.9 | 74.9 | 74.8 |
| Amine equivalent | 263 | 261 | 266 | 278 | 290 | 356 |

(continued)

| Properties NCO% NCO%(NV100%) | 16.0 21.3 | 16.1 21.2 | 15.8 20.5 | 15.1 20.5 | 14.5 19.3 | 11.8 15.8 |
|---|---|---|---|---|---|---|
| Trimolecular product area percentage (%) | 88 | 80 | 67 | 55 | 50 | 30 |
| * Reference Example | | | | | | |

[Table 2]

| No. | Production Example 7 | Production Example 8 | Production Example 9 |
|---|---|---|---|
| Alcohol modification agent | 1,3-BG | 1,3-BG | IBA |
| Alcohol modification percentage (wt%) | 1.96 | 1.96 | 1.96 |
| Additive Amount(phr) BHT JPP-100 | 0.025 0.05 | 0.025 0.05 | 0.025 0.05 |
| Reaction Conditions(urethane-formation) R(NCO/OH) Reaction temperature(°C) Reaction Time(min) | 24 75 120 | 24 75 120 | 39.4 75 120 |
| Catalyst Type Added Amount(phr) Added Amount solid content base(phr) | TBAOH 0.040 0.015 | TBAOH 0.064 0.024 | TBAOH 0.040 0.015 |
| Reaction Conditions(isocyanurate-formation) Reaction Start Temperature(°C) Maximum Temperature Reached(°C) Reaction Time(min) | 60 71 390 | 60 63 190 | 60 74 330 |
| Amine equivalent at the time of reaction start NCO% at the time of reaction start | 96.0 43.8 | 96.0 43.8 | 96.0 43.8 |
| Amine equivalent after urethane-formation reaction NCO% after urethane-formation reaction | 101.4 41.4 | 101.7 41.3 | 99.5 42.2 |
| Amine equivalent after isocyanurate-formation reaction NCO% after isocyanurate-formation reaction | 146.0 28.8 | 127.5 33.0 | 142.5 29.5 |
| Isocyanate group conversion rate(%) Urethane conversion rate(%) Trimer conversion rate(%) | 34.2 5.3 28.9 | 24.7 5.6 19.1 | 32.6 3.5 29.1 |
| Distillation yield(%) Alcohol modification percentage (wt%) Urethane-formation percentage(mol%) | 60.0 3.27 4.17 | 44.7 4.38 4.17 | 59.2 3.31 2.54 |
| Properties Solid content (%) Amine equivalent | 74.6 300 | 74.6 301 | 75.1 284 |

(continued)

| No. | Production Example 7 | Production Example 8 | Production Example 9 |
|---|---|---|---|
| NCO% | 14.0 | 14.0 | 14.8 |
| NCO%(NV100%) | 18.8 | 18.7 | 19.7 |
| Trimolecular product area percentage (%) | 39 | 51 | 38 |

The abbreviations in Tables are described below.
TBAOH: solution of tetrabutylammonium hydroxide, 37% methanol solution 1,3-BG: 1,3-butanediol
IBA: isobutanol (also called: isobutylalcohol)

Preparation Examples 1 to 35 and Comparative Preparation Examples 1 to 10

The isocyanurate derivative of xylylenediisocyanate produced in Production Examples and commercially available polyisocyanates were blended with the combinations and formulations shown in Tables 3 to 7, thereby producing curing agents. In the tables, the blending mass ratio is shown based on the solid content (solid content ratio).

The solid content concentration of the curing agent was adjusted in accordance with the evaluation method described later.

To be specific, the curing agent used in the solvent-based test (heat/non-heat-mature retort test, acid resistance retort test, and content resistance test described later) was diluted with ethyl acetate so that its solid content concentration was 75 mass%.

Vestanat T 1890/100 (isophorone diisocyanate trimer, manufactured by Evonik Japan Co., Ltd.) of Preparation Example 29 has a solid content concentration of 100 mass%, and this is difficult to be diluted with ethyl acetate. Thus, in Preparation Example 29, a curing agent was prepared so that the final solid content concentration was 75 mass% by adding Vestanat T 1890/100 to the isocyanurate derivative of xylylenediisocyanate of Production Example 4, and thereafter dissolved with ethyl acetate while heating.

The curing agent used for the no-solvent test (no-solvent adhesion retort test described later) was used as is without being diluted with a solvent.

Preparation Example 31 (preparation of main component A)

•Production of polyol A1

488.3 parts by mass of isophthalic acid, 137.7 parts by mass of adipic acid, 203.9 parts by mass of ethylene glycol, 219.0 parts by mass of neopentyl glycol, 290.5 parts by mass of 1,6-hexanediol, and 0.4 parts by mass of zinc acetate were subjected to esterification reaction under nitrogen flow at 180 to 220°C, and predetermined amounts of water and glycol were distilled off, thereby producing polyesterpolyol having a number average molecular weight of about 5000. The entire amount was dissolved in 800.0 g of ethyl acetate to produce a solution having a solid content (NV) of 60%, thereby producing polyol A1.

•Production of polyol A2

To 805.8 parts by mass of polyol A1 produced as described above, 16.54 parts by mass of 3-isocyanatomethyl-3,5,5-trimethylcyclohexylisocyanate and 0.25 parts by mass of tin octylate were added in a nitrogen atmosphere, and the mixture was subjected to urethane-forming reaction at 77 to 80°C for 4 hours. After disappearance of isocyanate groups was confirmed, 177.66 parts by mass of ethyl acetate was added thereto to produce a solution having a solid content of 50%, thereby producing polyol A2.

•Production of polyol A3

488.3 parts by mass of isophthalic acid, 137.7 parts by mass of adipic acid, 203.9 parts by mass of ethylene glycol, 219.0 parts by mass of neopentyl glycol, 290.5 parts by mass of 1,6-hexanediol, and 0.4 parts by mass of zinc acetate were subjected to esterification reaction under nitrogen flow at 180 to 220°C, and predetermined amounts of water and glycol were distilled off, thereby producing polyesterpolyol having a number average molecular weight of about 5000. The polyesterpolyol was cooled to 150°C, and thereafter 7.85 parts by mass of trimellitic anhydride was added thereto to cause reaction for 2 hours. The entire amount was dissolved in 805.2 parts by mass of ethyl acetate to produce a solution having a solid content of 60%, thereby producing polyol A3.

• Production of main component A

600.0 parts by mass of polyol A2, 333.3 parts by mass of polyol A3, 0.15 parts by mass of phosphoric acid (manufactured by Wako Pure Chemical Industries, Ltd.), 0.3 parts by mass of aminosilane (manufactured by Shin-Etsu Chemical Co., Ltd., trade name KBM 603), 4.5 parts by mass of epoxysilane (manufactured by Shin-Etsu Chemical Co., Ltd., trade name KBM 403), and 71.62 parts by mass of ethyl acetate were mixed to produce a solution having a solid content of 50%, thereby preparing main component A.

Preparation Example 32 (preparation of curing agent A)

A 1-liter four-neck flask equipped with a mixer, a thermometer, a condenser, and a nitrogen gas inlet tube was charged with 700 parts by mass of 1,3-xylylenediisocyanate (manufactured by Mitsui Chemicals, Inc., m-XDI), 27.6 parts by mass of isobutylalcohol, 0.36 parts by mass of (2-ethylhexyl) phosphite, and 0.36 parts by mass of pentaerythritoltetrakis [3-(3,5-di-t-butyl-4-hydroxyphenyl) propionate] in a nitrogen atmosphere, and the mixture was subjected to urethane-forming reaction at 75°C for 3.5 hours.
Then, 0.10 parts by mass of bismuth octylate (catalyst) was added to the reaction solution, and the mixture was subjected to allophanate formation reaction at 90°C for 24 hours. After confirming that the conversion from the urethane bond to the allophanate bond was almost completed, 0.10 parts by mass of orthotoluenesulfonamide was added to terminate the allophanate formation reaction.
Unreacted isobutylalcohol and 1,3-xylylenediisocyanate were removed from the produced reaction solution with a thin film distillation device (degree of vacuum: 0.05kPa, temperature 150°C), thereby producing curing agent A composed of allophanate derivative of xylylenediisocyanate.

Preparation example 33 (preparation of main component B)

• Preparation of polyol B1

588.1 g of isophthalic acid, 752.24 g of 1,3-butanediol, and 440.22 g of neopentyl glycol were subjected to esterification reaction under nitrogen flow at 180 to 220°C, and after a predetermined amount of water was distilled off, 258.66 g of adipic acid, 357.98 g of sebacic acid, and 0.08 g of titaniumtetrabutoxide were added thereto to subject the mixture to esterification reaction at 180 to 220°C, thereby producing polyester polyol B1 having a number average molecular weight of about 500.

• Production of main component B

750 g of polyol B1 and 57.6 g of trimellitic anhydride were subjected to reaction under nitrogen flow at 120 to 150°C for 3 hours, and after cooling to 60°C, 171.4 g of ACTCOL T-700 (polypropylenepolyol, manufactured by Mitsui Chemicals), 1.0 g of phosphoric acid, and 20.0 g of epoxysilane were added and mixed thereto, thereby producing resin having a solid content of 100%, i.e., main component B.

Examples 1 to 35 and Comparative Examples 1 to 10

The curing agent produced in Preparation Examples and Comparative Preparation Examples and the main component suitable for the tests below were mixed in accordance with the blending ratio (mass ratio) as shown in Tables 3 to 7, thereby producing a laminate adhesive.
In the Tables, the blending ratio (main component/curing agent blending ratio) in the solvent-based test (heat/non-heat-mature retort test, acid resistance retort test, and content resistance test) is a mass ratio of the main component containing solvent to the curing agent containing solvent. The blending ratio (main component/curing agent blending ratio) in the no-solvent test (no-solvent adhesion retort test described later) is a mass ratio of the main component containing no solvent to the curing agent containing no solvent.

Physical property evaluation

1) Heat/non-heat-mature retort test

For the main component, main component A produced in Preparation Example 31 was used. The main component and the curing agent produced in Preparation Examples and Comparative Preparation Examples were blended, thereby producing a laminate adhesive.

A composite film composed of 4 layers of polyethylene terephthalate film (thickness 12 $\mu$m)/nylon film (thickness 15 $\mu$m)/aluminum foil (thickness 9 $\mu$m)/cast polypropylene film (thickness 60 $\mu$m: One-side corona treated) was produced using the laminate adhesive.

That is, the laminate adhesive (curing agent and main component were blended) was applied, first to a polyethylene terephthalate film under normal temperature, and after the solvent was volatilized, the surface to which the laminate adhesive was applied was bonded to a nylon film having both sides subjected to corona treatment. Then, on the other side of the nylon film of the 2 layers of the composite film, in the same manner as described above, the laminate adhesive of Examples and Comparative Examples was applied. After the solvent was volatilized, the surface to which the laminate adhesive was applied was bonded to the aluminum foil. Then, on the other side of the aluminum foil of the 3 layer composite film, in the same manner as described above, the laminate adhesive of Examples and Comparative Examples was applied. After the solvent was volatilized, the surface to which the laminate adhesive was applied was bonded to the corona treated surface of the cast polypropylene film.

In the above-described bonding step, bonding was performed by further diluting the laminate adhesive (curing agent and main component were blended) suitably with ethyl acetate, so that the applied amount after the solvent volatilization was about 3.3 g/m$^2$.

Thereafter, the produced 4 layer composite film was matured at 40°C (heated) for 4 days, or at 24°C (non heated) for 4 days, thereby curing the laminate adhesive.

The laminate film produced as described above was subjected to measurement for peel strength between nylon/aluminum foil and between aluminum foil/cast polypropylene film in conformity with JIS K 6854-3(1999) at a constant temperature bath of 120°C and 24°C, a width 15 mm, and a tensile speed of 300 mm/min.

Then, using the produced laminate film, a bag having a size of 9 × 13 cm was produced, and the bag was charged with 150 g of contents: table vinegar/salad oil/ketchup mixture at a volume ratio of 1/1/1. The bag was placed on a tray having a size of 210 × 520 × 105 mm, and after subjecting the bag to hot water sterilization at 135°C for 20 minutes, and 8 rotations per minute under pressure of 0.35MPa, delamination between layers at the corner portions of the bag was checked. Three bags were tested per one type of sample.

Then, the peel strength between nylon/aluminum foil and between aluminum foil/cast polypropylene film after hot water sterilization test was measured in conformity with JIS K 6854-3(1999)) at 24°C, a width of 15 mm, and a tensile speed of 300 mm/min. Appearance after the hot water sterilization test was evaluated visually.

2) Acid resistance retort test

For the main component, TAKELAC A-505 (main ingredients: polyesterpolyurethane polyol, solid content concentration 50 mass%, solvent: ethyl acetate, commercially available product, manufactured by Mitsui Chemicals, Inc.) was used. The main component and the curing agent produced in Preparation Examples and Comparative Preparation Examples were mixed, thereby producing a laminate adhesive.

On the other side of the aluminum foil of the composite film in which polyethylene terephthalate film (thickness 12 $\mu$m)/aluminum foil (thickness 9 $\mu$m) was bonded with a laminate adhesive (manufactured by Mitsui Chemicals, Inc.) in advance, a laminate adhesive (in which curing agent and main component were blended) was applied under normal temperature. After the solvent was volatilized, the surface to which the laminate adhesive was applied was bonded to the corona treated surface of the cast polypropylene film (60 $\mu$m). Thereafter, the 3 layer composite film was matured at 50°C for 2 days to cure the laminate adhesive.

In the above-described bonding step, the laminate adhesive (in which curing agent and main component were blended) was further suitably diluted with ethyl acetate, and bonding was performed so that the amount applied after solvent volatilization was about 3.3 g/m$^2$.

Then, using the produced laminate film, a bag having a size of 9 cm × 13 cm was produced, and the bag was charged with 150 g of table vinegar. The bag was placed on a tray of 210 × 520 × 105 mm, and after subjecting the bag to hot water sterilization at 135°C for 20 minutes, 8 rotations per minute, under a pressure of 0.35MPa, the bag was stored at 60°C for 2 weeks.

Before and after hot water sterilization test, and after storage at 60°C for 2 weeks, the adhesive strength between the aluminum foil/cast polypropylene film was measured in conformity with JIS K 6854-3(1999) at 24°C, a width of 15 mm, and a tensile speed of 300 mm/min.

3) Content resistance test

For the main component, TAKELAC A-626 (main ingredients: polyesterpolyol, solid content concentration 60 mass%, solvent: ethyl acetate, commercially available product, manufactured by Mitsui Chemicals, Inc.) was used. The main component and the curing agent produced in Preparation Examples and Comparative Preparation Examples were mixed, thereby producing a laminate adhesive.

The produced laminate adhesive was applied on the corona treated surface of a nylon film (thickness 15 $\mu$m) having its one side corona treated, and after the solvent was volatilized, the surface to which the laminate adhesive was applied was bonded to a cast polyethylene film (thickness 130 $\mu$m). The produced laminate was matured at 40°C for 4 days, thereby curing the laminate adhesive.

In the above-described bonding step, the laminate adhesive (in which curing agent and main component were blended) was further suitably diluted with ethyl acetate, and bonding was performed so that the amount applied after solvent volatilization was about 3.3 g/m$^2$.

Then, using the produced laminate film, a bag having a size of 6.5 cm $\times$ 17.5 cm was produced, and the bag was charged with 30 g of a commercially available conditioner (Lux super damage repair, UNILEVER PLC), and stored at 50°C for 2 weeks.

The peel strength between the nylon film and cast polyethylene film was measured before and after the storage in conformity with JIS K 6854-3(1999) at 24°C, a width of 15 mm, and a tensile speed of 300 mm/min. The strength at the heat seal portion was also measured.

4) No-solvent adhesion retort test

For the main component, main component B produced in Preparation Example 33 was used. The main component and the curing agent produced in Preparation Examples and Comparative Preparation Examples were mixed, thereby producing a laminate adhesive.

On the other side of the aluminum foil of the composite film in which a polyethylene terephthalate film (thickness 12 $\mu$m)/aluminum foil (thickness 9 $\mu$m) was bonded with a laminate adhesive (manufactured by Mitsui Chemicals, Inc.) in advance, a laminate adhesive (in which curing agent and main component were blended) was applied with a no-solvent laminator at 60 to 80°C. The surface to which the laminate adhesive was applied was bonded to the corona treated surface of the cast polypropylene film (thickness 60 $\mu$m) so that the amount applied was about 3.0 g/m$^2$. Thereafter, the 3 layer composite film was matured at 40°C for 4 days, thereby curing the laminate adhesive.

Then, using the produced laminate film, a bag having a size of 9 cm $\times$ 13 cm was produced, and the bag was charged with 150 g of a mixture of table vinegar/salad oil/ketchup at a volume ratio of 1/1/1. The bag was placed on a tray of 210 $\times$ 520 $\times$ 105 mm, and after subjecting the bag to hot water sterilization at 121°C for 30 minutes, under a pressure of 0.20MPa, the bag was stored at 50°C for 2 weeks.

Before and after hot water sterilization test, and after storage at 50°C for 2 weeks, the adhesive strength between the aluminum foil/cast polypropylene film was measured in conformity with JIS K 6854-3(1999) at 24°C, a width of 15 mm, a tensile speed of 300 mm/min.

The details of the films used in the above-described tests are shown below. Polyethylene terephthalate film: manufactured by TOYOBO CO., LTD. ester film E 5102

Both sides corona treated nylon film: manufactured by Unitika Ltd., Emblem ONBC

Aluminum foil: manufactured by TOYO ALUMINIUM K.K. aluminum foil C

Cast polypropylene film: manufactured by Mitsui Chemicals Tohcello. Inc. CP RXC-22

One-side corona treated nylon film: manufactured by Unitika Ltd., Emblem ON Cast polyethylene film: manufactured by manufactured by Mitsui Chemicals Tohcello. Inc. T.U.X FC-D

[Table 3]

| No. | | | Comp. Example 1 | Comp. Example 2 | Comp. Example 3 | Example 1 * | Example 2 | Example 3 | Example 4 | Example 5 | Example 6 | Example 7 | Example 8 |
|---|---|---|---|---|---|---|---|---|---|---|---|---|---|
| Curing agent | | No. | Comp. Preparation Example 1 | Comp. Preparation Example 2 | Comp. Preparation Example 3 | Preparation Example 1 | Preparation Example 2 | Preparation Example 3 | Preparation Example 4 | Preparation Example 5 | Preparation Example 6 | Preparation Example 7 | Preparation Example 8 |
| | | Isocyanurate derivative of XDI | - | - | - | Production Example 1 | Production Example 2 | Production Example 3 | Production Example 5 | Production Example 6 | Production Example 9 | Production Example 8 | Production Example 7 |
| | | Trimolecular product area percentage (%) | - | - | - | 88 | 80 | 67 | 50 | 30 | 38 | 51 | 39 |
| | | Other polyisocyanate | D-110N | D-110N | D-110N | - | - | - | - | - | - | - | - |
| | | | D-140N | D-140N | - | - | - | - | - | - | - | - | - |
| | | Mixed mass ratio(Upper column/Lower column column) | 1/1 | 3/1 | - | - | - | - | - | - | - | - | - |
| | | Isocyanurate derivative of XDI content (%) | 0 | 0 | 0 | 100 | 100 | 100 | 100 | 100 | 100 | 100 | 100 |
| Heated/nonheated maturing retort test | | | | | | | | | | | | | |
| Main component/Curing agent blending ratio | | | 9/1 | 9/1 | 10/1 | 13/1 | 13/1 | 13/1 | 12/1 | 10/1 | 12/1 | 11/1 | 11/1 |
| 40°C 4 days maturing | Strength under 120°C | NY/AL | 3.5N | 2.9N | 2.6N | 2.8N | 3.0N | 3.5N | 3.4N | 3.1N | 3.2N | 3.4N | 3.2N |
| | | AL/CPP | 3.5N | 3.3N | 2.9N | 2.9N | 3.3N | 3.2N | 3.4N | 3.5N | 3.2N | 3.3N | 2.8N |
| | Before hot water sterilization test | NY/AL | 5.4N | 4.6N | 4.2N | 4.6N | 5.2N | 5.5N | 5.2N | 4.8N | 5.4N | 5.2N | 5.3N |
| | | AL/CPP | 10.9N | 10.1N | 9.8N | 11.1N | 10.8N | 11.9N | 11.4N | 10.8N | 11.8N | 11.2N | 10.8N |
| | After hot water sterilization test | NY/AL | 5.6N | 4.4N | 4.0N | 4.7N | 5.0N | 5.4N | 4.8N | 4.4N | 4.9N | 6.4N | 5.5N |
| | | AL/CPP | 6.5N | 6.5N | 6.1N | 6.3N | 6.1N | 3.8N | 6.2N | 6.5N | 6.5N | 6.4N | 5.7N |
| | | Delaminition | Not occurred | Not occurred | Occurred (1/8) | Not occurred | Not occurred | Not occurred | Not occurred | Not occurred | Not occurred | Not occurred | Not occurred |

EP 3 115 430 B1

(continued)

| No. | | | Comp. Example 1 | Comp. Example 2 | Comp. Example 3 | Example 1 * | Example 2 | Example 3 | Example 4 | Example 5 | Example 6 | Example 7 | Example 8 |
|---|---|---|---|---|---|---|---|---|---|---|---|---|---|
| 24°C 4 days maturing | Strength under 120°C | NY/AL | 0.8N | 1.3N | 0.8N | 2.1N | 2.4N | 2.8N | 2.0N | 2.4N | 2.0N | 2.6N | 2.2N |
| | | AL/CPP | 2.1N | 2.7N | 2.2N | 2.8N | 3.0N | 3.7N | 3.0N | 3.3N | 3.5N | 3.3N | 3.2N |
| | Before hot water sterilization test | NY/AL | 3.2N | 2.9N | 4.4N | 3.8N | 4.0N | 4.3N | 4.1N | 3.7N | 4.1N | 4.1N | 4.1N |
| | | AL/CPP | 8.4N | 10.9N | 7.3N | 9.6N | 9.3N | 8.9N | 9.0N | 9.5N | 9.5N | 8.4N | 9.0N |
| | After hot water sterilization test | NY/AL | 4.0N | 3.9N | 5.4N | 5.0N | 5.3N | 5.4N | 5.2N | 5.0N | 5.3N | 3.8N | 4.6N |
| | | AL/CPP | 6.1N | 6.5N | 5.4N | 5.6N | 5.8N | 5.4N | 5.4N | 5.8N | 5.5N | 5.9N | 5.5N |
| | | Delamination | Occurred (1/2) | Occurred (1/4) | Occurred (1/4) | Occurred (1/8) | Not occurred | Not occurred | Not occurred | Not occurred | Not occurred | Not occurred | Not occurred |
| Acid resistance retort test | | | | | | | | | | | | | |
| Main component/Curing agent blending ratio | | | 9/1 | 9/1 | 10/1 | 13/1 | 13/1 | 13/1 | 12/1 | 10/1 | 12/1 | 11/1 | 11/1 |
| 50°C 2 days maturing | Before hot water sterilization test | AL/CPP | 9.2N | 8.4N | 4.7N | 3.7N | 3.6N | 4.0N | 6.8N | 3.8N | 8.0N | 7.0N | 7.5N |
| | After hot water sterilization test | AL/CPP | 5.2N | 4.8N | 4.5N | 6.9N | 7.4N | 7.4N | 8.2N | 7.2N | 8.0N | 6.6N | 6.5N |
| | After storage at 60°C for 2 weeks | AL/CPP | 8.0N | 7.7N | 4.2N | 6.7N | 7.0N | 7.3N | 7.0N | 6.5N | 7.2N | 7.0N | 7.5N |
| Content resistance test | | | | | | | | | | | | | |
| Main component/Curing agent blending ratio | | | 8/1 | 8/1 | 9/1 | 12/1 | 12/1 | 12/1 | 11/1 | 9/1 | 11/1 | 10/1 | 10/1 |

24

| No. | | | Comp. Example 1 | Comp. Example 2 | Comp. Example 3 | Example 1 * | Example 2 | Example 3 | Example 4 | Example 5 | Example 6 | Example 7 | Example 8 |
|---|---|---|---|---|---|---|---|---|---|---|---|---|---|
| 40°C 4 days maturing | Initial period | NY/PE | NY break | NY break | 17.0N | NY break | NY break | NY break | NY break | NY break | 15.7N | NY break | 16.2N |
| | | Seal portion | 70N | 65N | 79N | 73N | 74N | 76N | 80N | 79N | 78N | 82N | 83N |
| | After storage at 50°C for 2 weeks | NY/PE | 7.4N | 8.1N | 3.7N | 7.0N | 8.3N | 9.8N | 8.0N | 8.1N | 7.2N | 8.8N | 9.3N |
| | | Seal portion | 72N | 79N | 51N | 26N | 57N | 79N | 77N | 74N | 61N | 79N | 71N |
| No solvent adhesion retort test | | | | | | | | | | | | | |
| Main component/Curing agent blending ratio | | | - | - | - | - | - | - | - | - | 10/6.5 | 10/6 | - |
| 40°C 4 days maturing | Before hot water sterilization test | AL/CPP | - | - | - | - | - | - | - | - | 4.6N | 4.3N | - |
| | | Seal portion | - | - | - | - | - | - | - | - | 40N | 44N | - |
| | After hot water sterilization test | AL/CPP | - | - | - | - | - | - | - | - | 4.1N | 4.2N | - |
| | | Seal portion | - | - | - | - | - | - | - | - | 37N | 39N | - |
| | After storage at 50°C for 2 weeks | AL/CPP | - | - | - | - | - | - | - | - | 4.1N | 4.1N | - |
| | | Seal portion | - | - | - | - | - | - | - | - | 35N | 34N | - |
| * Reference Example | | | | | | | | | | | | | |

EP 3 115 430 B1

Table 4]

| No. | | | Comp. Example 4 | Example 9 | Example 10 | Comp. Example 5 | Example 11 | Example 12 | Comp. Example 6 | Example 13 | Example 14 | Comp. Example 7 |
|---|---|---|---|---|---|---|---|---|---|---|---|---|
| Curing agent | No. | | Comp. Preparation Example 4] | Preparation Example 9 | Preparation Example 10 | Comp. Preparation Example 5 | Preparation Example 11 | Preparation Example 12 | Comp. Preparation Example 6 | Preparation Example 13 | Preparation Example 14 | Comp. Preparation Example 7 |
| | Isocyanurate derivative of XDI | | - | Production Example 3 | Production Example 3 | - | Production Example 3 | Production Example 3 | - | Production Example 3 | Production Example 3 | - |
| | Trimolecular product area percentage(%) | | - | 67 | 67 | - | 67 | 67 | - | 67 | 67 | - |
| | Other polyisocyanate | | Curing agent A | Curing agent A | D-110N | D-110N | Curing agent A | D-110N | D-110N | Curing agent A | D-110N | D-110N |
| | | | - | - | - | Curing agent A | - | - | Curing agent A | - | - | Curing agent A |
| | Mixed mass ratio(Upper column/Lower column) | | - | 2/1 | 2/1 | 2/1 | 1/1 | 1/1 | 1/1 | 1/2 | 1/2 | 1/2 |
| | Isocyanurate derivative of XDI content(%) | | 0 | 67 | 67 | 0 | 50 | 50 | 0 | 33 | 33 | 0 |
| Heated/nonheated maturing retort test | | | | | | | | | | | | |
| Main component/Curing agent blending ratio | | | 11/1 | 12/1 | 12/1 | 10/1 | 12/1 | 11/1 | 11/1 | 12/1 | 11/1 | 11/1 |
| 40°C 4 days maturing | Strength under 120°C | NY/AL | 1.5N | 2.6N | 2.4N | 2.7N | 3.0N | 2.7N | 2.6N | 3.2N | 3.0N | 2.6N |
| | | AL/CPP | 1.5N | 29N | 3.0N | 3.0N | 3.1N | 32N | 3.2N | 3.4N | 3.0N | 3.1N |
| | Before hot water sterilization test | NY/AL | 5.6N | 5.8N | 5.0N | 5.2N | 5.5N | 4.9N | 5.4N | 5.9N | 4.7N | 5.7N |
| | | AL/CPP | 13.7N | 11.0N | 11.1N | 10.2N | 11.3N | 10.5N | 10.9N | 12.1N | 9.8N | 11.9N |
| | After hot water sterilization test | NY/AL | 5.8N | 4.4N | 3.5N | 5.7N | 5.5N | 5.5N | 5.4N | 6.2N | 5.3N | 5.7N |
| | | AL/CPP | 6.1N | 6.3N | 6.0N | 6.2N | 6.1N | 5.9N | 6.5N | 6.4N | 6.5N | 6.2N |
| | | Delamination | Occurred(> 1/2) | Not occurred | Not occurred | Occurred (1/6) | Not occurred | Not occurred | Not occurred | Not occurred | Not occurred | Not occurred |

(continued)

| No. | | | Comp. Example 4 | Example 9 | Example 10 | Comp. Example 5 | Example 11 | Example 12 | Comp. Example 6 | Example 13 | Example 14 | Comp. Example 7 |
|---|---|---|---|---|---|---|---|---|---|---|---|---|
| 24°C 4 days maturing | Strength under 120°C | NY/AL | 0.7N | 1.9N | 2.0N | 1.4N | 1.6N | 1.8N | 1.1N | 0.9N | 1.6N | 1.1N |
| | | AL/CPP | 0.8N | 2.7N | 33N | 2.9N | 2.6N | 2.8N | 2.5N | 1.5N | 3.3N | 2.6N |
| | Before hot water sterilization test | NY/AL | 3.6N | 3.9N | 3.1N | 3.7N | 4.0N | 3.5N | 3.9N | 3.7N | 3.6N | 4.0N |
| | | AL/CPP | 11.3N | 10.3N | 9.6N | 10.1N | 11.0P | 9.3N | 11.0N | 10.1N | 8.4N | 10.5N |
| | After hot water sterilization test | NY/AL | 3.7N | 5.4N | 4.6N | 3.0N | 4.9N | 5.0N | 4.4N | 3.9N | 5.0N | 4.5N |
| | | AL/CPP | 5.5N | 6.1N | 5.5N | 5.8N | 5.6N | 6.3N | 5.7N | 5.5N | 6.4N | 6.0N |
| | | Delamination | Occurred (1/4 ) | Not occurred | Not occurred | Occurred(> 1/2) | Not occurred | Not occurred | Occurred (1/4) | Not occurred | Occurred (1/10) | Occurred (1/4) |
| Acid resistance retort test | | | | | | | | | | | | |
| Main component/Curing agent blending ratio | | | 11/1 | 12/1 | 12/1 | 10/1 | 12/1 | 11/1 | 11/1 | 12/1 | 11/1 | 11/1 |
| 50°C 2 days maturing | Before hot water sterilization test | AL/CPP | 5.3N | 10.2N | 8.8N | 9.8N | 9.2N | 8.4N | 9.4N | 6.7N | 6.3N | 7.0N |
| | After hot water sterilization test | AL/CPP | 6.2N | 7.8N | 6.6N | 5.9N | 6.9N | 4.6N | 5.0N | 6.3N | 5.2N | 5.5N |
| | After storage at 60°C for 2 weeks | AL/CPP | 6.6N | 8.0N | 7.9N | 6.8N | 6.3N | 5.4N | 5.7N | 6.9N | 5.7N | 4.3N |
| Content resistance test | | | | | | | | | | | | |
| Main component/Curing agent blending ratio | | | 10/1 | 11/1 | 11/1 | 9/1 | 11/1 | 10/1 | 10/1 | 11/1 | 10/1 | 10/1 |

| No. | | | Comp. Example 4 | Example 9 | Example 10 | Comp. Example 5 | Example 11 | Example 12 | Comp. Example 6 | Example 13 | Example 14 | Comp. Example 7 |
|---|---|---|---|---|---|---|---|---|---|---|---|---|
| 40°C 4 days maturing | Initial period | NY/PE | 15.6N | 14.8N | NY break | NY break | 15.0N | 15.9N | 15.6N | 15.5N | 14.9N | 14.5N |
| | | Seal portion | 74N | 82N | 69N | 68N | 81N | 80N | 78N | 79N | 75N | 80N |
| | After 50°C storage at for 2 weeks | NY/PE | 4.8N | 9.8N | 7.6N | 6.5N | 8.0N | 6.5N | 4.3N | 7.3N | 6.1N | 3.5N |
| | | Seal portion | 46N | 75N | 83N | 77N | 75N | 48N | 25N | 78N | 55N | 32N |
| No solvent adhesion retort test | | | | | | | | | | | | |
| Main component/Curing agent blending ratio | | | 10/6.5 | - | - | - | - | - | - | - | - | - |
| 40°C 4 days maturing | Before hot sterilization water test | AL/CPP | 3.9N | - | - | - | - | - | - | - | - | - |
| | | Seal portion | 28N | - | - | - | - | - | - | - | - | - |
| | After hot sterilization water test | AL/CPP | 1.2N | - | - | - | - | - | - | - | - | - |
| | | Seal portion | 22N | - | - | - | - | - | - | - | - | - |
| | After 50°C 2 storage at for weeks | AL/CPP | Delamination | - | - | - | - | - | - | - | - | - |
| | | Seal portion | - | - | - | - | - | - | - | - | - | - |

EP 3 115 430 B1

[Table 5]

| No. | | | Ex. 15 * | Ex.16 | Ex.17 | Ex. 18 | Ex. 19 | Ex. 20 * | Comp. Ex. 8 |
|---|---|---|---|---|---|---|---|---|---|
| Curing agent | | No. | Preparation Ex. 15 Preparation Ex.15 | Preparation Ex. 16 Preparation Ex.15 | Preparation Ex. 17 | Preparation Ex. 18 Preparation Ex.15 | Preparation Ex. 19 Preparation Ex.15 | Preparation Ex. 20 | Comp. Preparation Ex. 8 |
| | | Isocyanurate derivative XDI | Production Ex. 1 | Production Ex. 2 | Production Ex. 4 | Production Ex. 4 | Production Ex. 4 | Production Ex. 4 | - |
| | | Trimolecular product area percentage(%) | 88 | 80 | 55 | 55 | 55 | 55 | - |
| | | Other polyisocyanate | D-140N | D-140N | D-140N | D-140N | D-140N | D-140N | D-140N |
| | | | - | - | - | - | - | - | - |
| | | Mixed mass ratio(Upper column/Lower column) | 1/2 | 1/2 | 2/1 | 1/2 | 1/4 | 1/8 | - |
| | | Isocyanurate derivative XDI content(%) | 33 | 33 | 67 | 33 | 20 | 11 | 0 |
| Heated/nonheated maturing retort test | | | | | | | | | |
| Main component/Curing agent blending ratio | | | 10/1 | 10/1 | 11/1 | 10/1 | 9/1 | 9/1 | 8/1 |
| 40℃ 4] days maturing | Strength under 120°C | NY/AL | 2.1N | 2.4N | 3.3N | 2.8N | 2.3N | 1.1N | 1.7N |
| | | AL/CPP | 3.2N | 3.3N | 3.3N | 3.3N | 3.5N | 3.3N | 2.8N |
| | Before hot water sterilization test | NY/AL | 4.1N | 4.3N | 5.2N | 4.4N | 3.8N | 3.6N | 3.3N |
| | | AL/CPP | 9.3N | 9.7N | 11.1N | 12.2N | 9.7N | 9.0N | 8.6N |
| | After hot water sterilization test | NY/AL | 5.5N | 5.4N | 6.2N | 5.8N | 6.2N | 4.4N | 5.8N |
| | | AL/CPP | 5.9N | 6.0N | 6.2N | 5.7N | 5.9N | 5.5N | 6.1N |
| | | Delamination | Not occurred | Not occurred | Not occurred | Not occurred | Not occurred | Not occurred | Not occurred |

| No. | | | Ex. 15 * | Ex.16 | Ex.17 | Ex. 18 | Ex. 19 | Ex. 20 * | Comp. Ex. 8 |
|---|---|---|---|---|---|---|---|---|---|
| 24°C 4 days maturing | Strength under 120°C | NY/AL | 1.1N | 1.6N | 1.8N | 1.5N | 1.3N | 0.4N | 0.3N |
| | | AL/CPP | 2.5N | 3.0N | 3.7N | 2.1N | 2.2N | 1.0N | 0.8N |
| | Before hot water sterilization test | NY/AL | 3.1N | 3.6N | 3.3N | 3.2N | 3.6N | 3.2N | 3.3N |
| | | AL/CPP | 6.6N | 6.4N | 8.0N | 6.0N | 5.2N | 4.8N | 4.8N |
| | After hot water sterilization test | NY/AL | 4.2N | 4.5N | 4.3N | 4.1N | 4.8N | 5.0N | 3.0N |
| | | AL/CPP | 6.1N | 5.8N | 5.9N | 6.0N | 5.7N | 5.9N | 6.0N |
| | | Delamination | Occurred(1/3)/3) | Not occurred | Not occurred | Not occurred | Not occurred | Occurred(> 1/2) | Occurred (Entirely) |
| Acid resistance retort test | | | | | | | | | |
| Main component/Curing agent blending ratio | | | 10/1 | 10/1 | 11/1 | 10/1 | 9/1 | 9/1 | 8/1 |
| 50°C 2 days maturing | Before hot water sterilization test | AL/CPP | 9.8N | 10.2N | 10.0N | 10.8N | 8.8N | 7.8N | 7.4N |
| | After hot water sterilization test | AL/CPP | 5.5N | 5.5N | 6.5N | 5.8N | 5.3N | 4.8N | 4.5N |
| | After storage at 60°C for 2 weeks | AL/CPP | 6.1N | 6.5N | 8.6N | 7.1N | 6.6N | 5.4N | 4.4N |
| Content resistance test | | | | | | | | | |
| Main component/Curing agent blending ratio | | | 9/1 | 9/1 | 10/1 | 9/1 | 8/1 | 8/1 | 7/1 |

EP 3 115 430 B1

(continued)

(continued)

| No. | | | Ex. 15 * | Ex.16 | Ex.17 | Ex. 18 | Ex. 19 | Ex. 20 * | Comp. Ex. 8 |
|---|---|---|---|---|---|---|---|---|---|
| 40°C 4] days maturing | Initial period | NY/PE | 13.5N | NY break | NY break | NY break | 14.7N | 15.3N | NY break |
| | | Seal portion | 74N | 81N | 80N | 80N | 83N | 77N | 75N |
| | After storage at 50°C for 2 weeks | NY/PE | 7.4N | 8.0N | 8.5N | 7.8N | 7.0N | 6.6N | 6.3N |
| | | Seal portion | 75N | 80N | 80N | 76N | 73N | 68N | 73N |
| No solvent adhesion retort test | | | | | | | | | |
| Main component/Curing agent blending ratio | | | - | - | - | - | - | - | - |
| 40°C 4 days maturing | Before hot water sterilization test | AL/CPP | - | - | - | - | - | - | - |
| | | Seal portion | - | - | - | - | - | - | - |
| | After hot water sterilization test | AL/CPP | - | - | - | - | - | - | - |
| | | Seal portion | - | - | - | - | - | - | - |
| | After storage at 50°C for 2 weeks | AL/CPP | - | - | - | - | - | - | - |
| | | Seal portion | - | - | - | - | - | - | - |
| * Reference Examples 64 | | | | | | | | | |

EP 3 115 430 B1

31

[Table 6]

| No. | | | Ex.21 * | Ex. 22 | Ex.23 | Ex. 24 | Ex. 25 | Ex. 26 | Comp. Ex. 9 | Ex. 27 | Ex. 28 | Ex. 29 | Ex. 30 |
|---|---|---|---|---|---|---|---|---|---|---|---|---|---|
| Curing agent | No. | | Preparation Ex. 21 | Preparation Ex. 22 | Preparation Ex. 23 | Preparation Ex. 24 | Preparation Ex. 25 | Preparation Ex. 26 | Comp. Preparation Ex. | Preparation Ex. 27 | Preparation Ex. 28 | Preparation Ex. 29 | Preparation Ex. 30 |
| | Isocyanurate derivative of XDI | | Production Ex. 1 | Production Ex. 2 | Production Ex. 4 | Production Ex. 4 | Production Ex. 4 | Production Ex. 4 | - | Production Ex. 4 | Production Ex. 4 | Production Ex. 4 | Production Ex. 4 |
| | Trimolecular product area percentage(%) | | 88 | 80 | 55 | 55 | 55 | 55 | - | 55 | 55 | 55 | 55 |
| | Other polyisocyanate | | D-170N | D-170N | D-170N | D-170N | D-170N | D-170N | D-170N | D-165N | D-160N | T1890 | D-170N |
| | | | - | - | - | - | - | - | - | - | - | - | D-140N |
| | Mixed mass ratio(Upper column/Lower column) | | 1/2 | 1/2 | 2/1 | 1/2 | 1/4 | 1/8 | - | 1/2 | 1/2 | 2/1 | 1/1/1 |
| | Isocyanurate derivative of XDI content(%) | | 33 | 33 | 67 | 33 | 20 | 11 | 0 | 33 | 33 | 67 | 33 |
| Heated/nonheated maturing retort test | | | | | | | | | | | | | |
| Main component/Curing agent blending ratio | | | 13/1 | 13/1 | 13/1 | 13/1 | 13/1 | 13/1 | 13/1 | 14/1 | 11/1 | 14/1 | 11/1 |
| 40°C 4 days maturing | Strength under 120°C | NY/AL | 3.0N | 3.3N | 3.IN | 3.0N | 3.5N | 3.2N | 3.1N | 3.5N | 3.2N | 2.8N | 3.4N |
| | | AL/CPP | 3.0N | 3.2N | 3.5N | 3.1N | 3.2N | 2.9N | 3.5N | 3.3N | 3.3N | 3.7N | 3.1N |
| | Before hot water sterilization test | NY/AL | 5.7N | 5.3N | 4.8N | 5.3N | 5.4N | 4.9N | 5.3N | 5.0N | 4.7N | 3.9N | 5.1N |
| | | AL/CPP | 11.3N | 11.9N | 11.4N | 10.9N | 11.7N | 12.0N | 12.3N | 11.5N | 10.8N | 8.7N | 10.0N |
| | After hot water sterilization test | NY/AL | 5.4N | 5.6N | 5.6N | 4.8N | 5.4N | 3.4N | 5.1N | 5.5N | 5.8N | 5.1N | 5.2N |
| | | AL/CPP | 6.4N | 6.1N | 6.3N | 5.7N | 6.0N | 6.4N | 7.2N | 6.3N | 6.4N | 6.4N | 6.0N |
| | | Delamination | Not occurred | Not occurred | Not occurred | Not occurred | Not occurred | Not occurred | Not occurred | Not occurred | Not occurred | Not occurred | Not occurred |

(continued)

| No. | | | Ex. 21 * | Ex. 22 | Ex.23 | Ex. 24 | Ex. 25 | Ex. 26 | Comp. Ex. 9 | Ex. 27 | Ex. 28 | Ex. 29 | Ex. 30 |
|---|---|---|---|---|---|---|---|---|---|---|---|---|---|
| 24°C 4 days maturing | Strength under 120°C | NY/AL | 1.8N | 2.1N | 2.0N | 2.4N | 2.0N | 1.7N | 1.6N | 2.5N | 2.3N | 2.2N | 2.2N |
| | | AL/CPP | 3.5N | 3.2N | 3.3N | 3.4N | 3.3N | 3.2N | 3.0N | 3.3N | 3.1N | 3.1N | 3.0N |
| | Before hot water sterilization test | NY/AL | 3.7N | 4.1N | 3.3N | 3.8N | 4.2N | 3.8N | 4.1N | 4.4N | 3.8N | 3.4N | 3.9N |
| | | AL/CPP | 9.9N | 9.7N | 9.7N | 9.8N | 10.4N | 10.7N | 10.2N | 11.2N | 10.3N | 8.9N | 9.3N |
| | After hot water sterilization test | NY/AL | 4.9N | 5.2N | 3.6N | 5.0N | 5.6N | 5.2N | 5.6N | 5.4N | 5.7N | 4.5N | 5.0N |
| | | AL/CPP | 5.9N | 5.6N | 6.2N | 6.3N | 5.8N | 5.9N | 6.1N | 5.9N | 5.7N | 6.0N | 5.7N |
| | | Delamination | Occurred (1/8) | Not occurred | Not occurred | Not occurred | Not occurred | Not occurred | Occurred (1/8) | Not occurred | Not occurred | Not occurred | Not occurred |
| Acid resistance retort test | | | | | | | | | | | | | |
| Main component/Curing agent blending ratio | | | 13/1 | 13/1 | 13/1 | 13/1 | 13/1 | 13/1 | 13/1 | 14/1 | 11/1 | 14/1 | 11/1 |
| 50°C2 days maturing | Before hot water sterilization test | AL/CPP | 9.5N | 9.3N | 9.2N | 9.6N | 9.8N | 10.3N | 10.8N | 10.0N | 10.3N | 9.7N | 9.3N |
| | After hot water sterilization test | AL/CPP | 3.4N | 4.0N | 4.7N | 3.4N | 3.3N | 3.5N | 4.0N | 5.2N | 5.6N | 4.5N | 6.3N |
| | After storage at 60°C for 2 weeks | AL/CPP | 6.6N | 6.7N | 7.4N | 7.0N | 6.5N | 4.2N | 4.2N | 6.8N | 6.3N | 7.0N | 6.8N |
| Content resistance test | | | | | | | | | | | | | |
| Main component/Curing agent blending ratio | | | 12/1 | 12/1 | 12/1 | 12/1 | 12/1 | 12/1 | 12/1 | 12/1 | 10/1 | 12/1 | 10/1 |
| 40°C 4 days maturing | Initial period | NY/PE | 15.5N | 14.3N | NY break | NY break | 15.8N | 14.5N | 14.8N | 15.1N | 14.9N | NY break | NY break |
| | | Seal portion | 76N | 77N | 73N | 78N | 75N | 77N | 79N | 75N | 77N | 83N | 83N |
| | After storage at 50°C for 2 weeks | NY/PE | 6.0N | 6.4N | 7.2N | 6.5N | 5.8N | 6.3N | 6.3N | 7.0N | 6.8N | 8.9N | 9.2N |
| | | Seal portion | 77N | 78N | 83N | 81N | 76N | 68N | 70N | 74N | 80N | 84N | 82N |

(continued)

| No. | | | Ex.21 * | Ex. 22 | Ex.23 | Ex. 24 | Ex. 25 | Ex. 26 | Comp. Ex. 9 | Ex. 27 | Ex. 28 | Ex. 29 | Ex. 30 |
|---|---|---|---|---|---|---|---|---|---|---|---|---|---|
| No solvent adhesion retort test | | | | | | | | | | | | | |
| Main component/Curing agent blending ratio | | | - | - | - | - | - | - | 10/5 | - | - | - | - |
| 40°C4 days maturing | Before hot water sterilization test | AL/CPP | - | - | - | - | - | - | 4.0N | - | - | - | - |
| | | Seal portion | - | - | - | - | - | - | 40N | - | - | - | - |
| | After hot water sterilization test | AUCPP | - | - | - | - | - | - | 1.2N | - | - | - | - |
| | | Seal portion | - | - | - | - | - | - | 26N | - | - | - | - |
| | After storage at 50°C for 2 weeks | AL/CPP | - | - | - | - | - | - | 1.9N | - | - | - | - |
| | | Seal portion | - | - | - | - | - | - | 22N | - | - | - | - |
| * Reference Example | | | | | | | | | | | | | |

[Table 7]

| | | Example 31 | Example 32 | Example 33 | Example 34 | Example 35 | Comparative Example10 |
|---|---|---|---|---|---|---|---|
| No. | | Preparation Example31 | Preparation Example32 | Preparation Example33 | Preparation Example34 | Preparation Example35 | Comparative Preparation Example 10 |
| Curing agent | Isocyanurate derivative of XDI | Production Example 8 | Production Example 8 | Production Example 8 | Production Example 8 | Production Example 8 | - |
| | Trimolecular product area percentane(%) | 51 | 51 | 51 | 51 | 51 | - |
| | Other polyisocyanate | D-170N | D-170N | D-170N | D-170N | D-165N | D-165N |
| | Mixed mass ratio(Upper column/Lower column) | 1/1 | 1/2 | 1/4 | 1/8 | 1/1 | - |
| | Isocyanurate derivative of XDI content(%) | 50 | 33 | 20 | 11 | 50 | 0 |
| Heated/nonheated maturing retort test | | | | | | | |
| Main component/Curing agent blending ratio | | - | - | - | - | - | - |
| 40°C 4 days maturing | Strength under 120°C NY/AL | - | - | - | - | - | - |
| | Strength under 120°C AL/CPP | - | - | - | - | - | - |
| | Before hot water sterilization test NY/AL | - | - | - | - | - | - |
| | Before hot water sterilization test AL/CPP | - | - | - | - | - | - |
| | After hot water sterilization test NY/AL | - | - | - | - | - | - |
| | After hot water sterilization test AL/CPP | - | - | - | - | - | - |
| | Delamination | - | - | - | - | - | - |

| No. | | | Example 31 | Example 32 | Example 33 | Example 34 | Example 35 | Comparative Example 10 |
|---|---|---|---|---|---|---|---|---|
| 24°C 4 days maturing | Strength under 120°C | NY/AL | - | - | - | - | - | - |
| | | AL/CPP | - | - | - | - | - | - |
| | Before hot water sterilization test | NY/AL | - | - | - | - | - | - |
| | | AL/CPP | - | - | - | - | - | - |
| | After hot water sterilization test | NY/AL | - | - | - | - | - | - |
| | | AL/CPP | - | - | - | - | - | - |
| | | Delamination | - | - | - | - | - | - |
| Acid resistance retort test | | | | | | | | |
| Main component/Curing agent blending ratio | | | - | - | - | - | - | - |
| 50°C 2 days maturing | Before hot water sterilization test | AL/CPP | - | - | - | - | - | - |
| | After hot water sterilization test | AL/CPP | - | - | - | - | - | - |
| | After storage at 60°C for 2 weeks | AL/CPP | - | - | - | - | - | - |
| Content resistance test | | | | | | | | |
| Main component/Curing agent blending ratio | | | - | - | - | - | - | - |
| 40°C 4 days maturing | Initial period | NY/PE | - | - | - | - | - | - |
| | | Seal portion | - | - | - | - | - | - |
| | After storage at 50°C for 2 weeks | NY/PE | - | - | - | - | - | - |
| | | Seal portion | - | - | - | - | - | - |
| No solvent adhesion retort test | | | | | | | | |
| Main component/Curing agent blending ratio | | | 10/5.5 | 10/5.5 | 10/5.5 | 10/5.5 | 10/5.5 | 10/5 |

| No. | | | Example 31 | Example 32 | Example 33 | Example 34 | Example 35 | Comparative Example 10 |
|---|---|---|---|---|---|---|---|---|
| 40°C 4 days maturing | Before hot water sterilization test | AL/CPP | 5.0N | 4.8N | 4.7N | 4.6N | 5.3N | 4.6N |
| | | Seal portion | 43N | 43N | 41N | 40N | 42N | 42N |
| | After hot water sterilization test | AL/CPP | 3.9N | 4.0N | 2.0N | 1.4N | 4.6N | 1.1N |
| | | Seal portion | 37N | 37N | 27N | 30N | 40N | 29N |
| | After storage at 50°C for 2 weeks | AL/CPP | 3.6N | 3.2N | 1.8N | 1.9N | 3.5N | 2.7N |
| | | Seal portion | 36N | 35N | 30N | 22N | 35N | 26N |

The abbreviations in Tables are shown below.

D-110N: TAKENATE D-110N, trimethylolpropane modified xylylenediisocyanate, solid content concentration: 75 mass%, manufactured by Mitsui Chemicals, Inc.
D-140N: TAKENATE D-140N, trimethylolpropane modified isophoronediisocyanate, solid content concentration: 75 mass%, manufactured by Mitsui Chemicals, Inc.
D-160N: TAKENATE D-160N, trimethylolpropane modified hexamethylene diisocyanate, solid content 75 mass%, manufactured by Mitsui Chemicals, Inc.
D-165N: TAKENATE D-165N, biuret-modified hexamethylene diisocyanate, solid content concentration: 100 mass%, manufactured by Mitsui Chemicals, Inc.
D-170N: TAKENATE D-170N, trimer of hexamethylene diisocyanate, solid content concentration: 100 mass%, manufactured by Mitsui Chemicals, Inc.
T 1890: Vestanat T 1890/100, trimer of isophoronediisocyanate, solid content concentration: 100 mass%, manufactured by Evonik Japan Co., Ltd.
Curing agent A: allophanate derivative of xylylenediisocyanate, solid content concentration: 100 mass%, curing A agent produced in Preparation Example 32 NY: nylon
AL: aluminum foil
CPP: cast polypropylene film
PE: cast polyethylene film
Delamination: appearance evaluation. Shows occurrence or non-occurrence of delamination. "Not occurred" shows that no delamination was confirmed, and "Occurred" shows that delamination was confirmed. The numeral value following the "occurred" shows the proportion of the region (length) where delamination was confirmed relative to the length of heat seal portion of the sample.

While the illustrative embodiments of the present invention are provided in the above description, such is for illustrative purpose only and it is not to be construed as limiting in any manner. Modification and variation of the present invention that will be obvious to those skilled in the art is to be covered by the following claims.

Industrial Applicability

The laminate adhesive, the method for producing a laminate film in which the laminate adhesive was used, the laminate film produced by the method, and the retort pouch material including the laminate film of the present invention are suitably used in the field of wrapping field.

**Claims**

1. A laminate adhesive comprising:

   a curing agent including a polyisocyanate component and a main component including a polyol component, wherein the polyisocyanate component includes an isocyanurate derivative of xylylenediisocyanate, the content of the isocyanurate derivative of xylylenediisocyanate in the polyisocyanate component being 20 mass% or more, and
   in the chromatogram when the isocyanurate derivative of xylylenediisocyanate is subjected to gel permeation chromatograph measurement, the area percentage of a peak area having peak top between the polystyrene-based molecular weight of 400 to 1000 relative to the total peak area is 30% or more and 85% or less.

2. The laminate adhesive according to Claim 1, wherein
   in the chromatogram when the isocyanurate derivative of xylylenediisocyanate is subjected to gel permeation chromatograph measurement, the area percentage of a peak area having peak top between the polystyrene-based molecular weight of 400 to 1000 relative to the total peak area is 35% or more and 80% or less.

3. A method for producing a laminate film in which a plurality of films are allowed to adhere using the laminate adhesive according to Claim 1.

4. The method for producing a laminate film according to Claim 3, wherein the adhered film is matured without heating.

5. A laminate film comprising:

a plurality of films,
wherein the plurality of films are allowed to adhere using the laminate adhesive according to Claim 1.

6. A retort pouch material comprising the laminate film according to Claim 5.


**Patentansprüche**

1. Laminatklebstoff, umfassend:

   ein Härtungsmittel, das eine Polyisocyanatkomponente und eine Hauptkomponente, enthaltend eine Polyolkomponente, enthält,
   wobei die Polyisocyanatkomponente ein Isocyanuratderivat von Xylylendiisocyanat enthält, wobei der Gehalt des Isocyanuratderivats von Xylylendiisocyanat in der Polyisocyanatkomponente 20 Massen-% oder mehr beträgt, und
   wenn das Isocyanuratderivat von Xylylendiisocyanat einer Gelpermeationschromatographiemessung unterzogen wird, in dem Chromatogramm der Flächenprozentsatz einer Peakfläche mit einer Peakspitze zwischen dem Polystyrolbasierten Molekulargewicht von 400 bis 1000 relativ zur gesamten Peakfläche 30% oder mehr und 85% oder weniger beträgt.

2. Laminatklebstoff gemäß Anspruch 1, wobei
   wenn das Isocyanuratderivat von Xylylendiisocyanat einer Gelpermeationschromatographiemessung unterzogen wird, in dem Chromatogramm der Flächenprozentsatz einer Peakfläche mit einer Peakspitze zwischen dem Polystyrolbasierten Molekulargewicht von 400 bis 1000 relativ zur gesamten Peakfläche 35% oder mehr und 80% oder weniger beträgt.

3. Verfahren zur Herstellung eines Laminatfilms, bei dem mehrere Filme unter Verwendung des Laminatklebstoffs gemäß Anspruch 1 haften gelassen werden.

4. Verfahren zur Herstellung eines Laminatfilms gemäß Anspruch 3, wobei der anhaftende Film ohne Erhitzen gereift ist.

5. Laminatfolie, umfassend:

   mehrere Filme,
   wobei die mehreren Filme unter Verwendung des Laminatklebstoffs gemäß Anspruch 1 anhaften.

6. Retortenbeutelmaterial, umfassend die Laminatfolie gemäß Anspruch 5.


**Revendications**

1. Adhésif de lamination comprenant :

   un agent de durcissement incluant un composant polyisocyanate et un composant principal incluant un composant polyol,
   dans lequel le composant polyisocyanate inclut un dérivé isocyanurate de xylylènediisocyanate, la teneur du dérivé isocyanurate de xylylènediisocyanate dans le composant polyisocyanate étant de 20 % en masse ou plus, et
   dans le chromatogramme, lorsque le dérivé isocyanurate de xylylènediisocyanate est soumis à une mesure par chromatographie par perméation de gel, le pourcentage de surface d'une surface de pic ayant un sommet de pic dans une plage de 400 à 1000 de masse moléculaire en équivalent polystyrène par rapport à la surface de pic totale est de 30 % ou plus et de 85 % ou moins.

2. Adhésif de lamination selon la revendication 1, dans lequel
   dans le chromatogramme, lorsque le dérivé isocyanurate de xylylènediisocyanate est soumis à une mesure par chromatographie par perméation de gel, le pourcentage de surface d'une surface de pic ayant un sommet de pic dans une plage de 400 à 1000 de masse moléculaire en équivalent polystyrène par rapport à la surface de pic totale est de 35 % ou plus et de 80 % ou moins.

3. Procédé de production d'un film de lamination dans lequel on fait adhérer une pluralité de films au moyen de l'adhésif de lamination selon la revendication 1.

4. Procédé de production d'un film de lamination selon la revendication 3, dans lequel le film collé est amené à maturation sans chauffage.

5. Film de lamination comprenant :

   une pluralité de films,
   dans lequel on fait adhérer la pluralité de films au moyen de l'adhésif de lamination selon la revendication 1.

6. Matériau pour sachet stérilisable comprenant le film de lamination selon la revendication 5.

# EP 3 115 430 B1

## FIG. 1

Vial 37    Channel 410

Sample weight 0.0400

THF solvent GPC chart

Analysis date 2014/07/04 13:08:47 JST

### GPC result

| | Retention Time | MP | Mn | Mw | Dispersion degree | Area | Height | Start time | End time | %area | Division |
|---|---|---|---|---|---|---|---|---|---|---|---|
| 1 | 26.053 | 1460 | 1691 | 1772 | 1.047509 | 3408802 | 106910 | 24.317 | 26.183 | 12.27 | bV |
| 2 | 26.450 | 1098 | 1089 | 1100 | 1.009748 | 5704280 | 270272 | 26.183 | 26.750 | 20.54 | VV |
| 3 | 27.072 | 701 | 678 | 687 | 1.014404 | 18663747 | 955524 | 26.750 | 28.100 | 67.19 | Vv |

41

FIG. 2

Vial 64      Channel 410

Sample weight 0.0400

THF solvent GPC chart

Analysis date 2014/09/11 14 27 36 JST

## GPC result

| | Retention Time | MP | Mn | Mw | Dispersion degree | Area | Height | Start time | End time | %area | Division |
|---|---|---|---|---|---|---|---|---|---|---|---|
| 1 | 25.873 | 1618 | 2237 | 2568 | 1.147674 | 6550752 | 129358 | 22.300 | 25.950 | 25.52 | bV |
| 2 | 26.250 | 1236 | 1233 | 1249 | 1.012851 | 5941036 | 227228 | 25.950 | 26.533 | 23.14 | VV |
| 3 | 26.870 | 792 | 767 | 783 | 1.019958 | 13182155 | 602632 | 26.533 | 27.867 | 51.34 | VV |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003113359 A
- JP 2000154365 A
- JP 2002155260 A
- JP 2000351953 A
- JP S61129173 B